# FASCICULE DE BREVET EUROPEEN

(11) **EP 1 538 222 B1**
(45) Date de publication et mention de la délivrance du brevet: **02.02.2011**
(21) Numéro de dépôt: 04078201.3
(22) Date de dépôt: 15.01.1996
(51) Int. Cl.: C12Q 1/68, C07K 14/255

(54) **Oligonucléotides pour la détection de salmonella**
Oligonukleotide zur Detektion von Salmonella
Oligonucleotides for the detection of salmonella

(30) Priorité: 16.01.1995 FR 9500410
(43) Date de publication de la demande: 08.06.2005
(62) Demande divisionnaire de: 96400098.8
(73) Titulaire: INSTITUT PASTEUR, 75724 Paris Cedex 15 (FR); INSTITUT NATIONAL DE LA SANTE ET DE LA RECHERCHE MEDICALE (INSERM), 75654 Paris Cédex 13 (FR)
(72) Inventeur: Popoff, Michel Yvan, 66140 Canet-en-Roussillon (FR); Le Guern Fellous, Muriel, 92500 Rueil Malmaison (FR)
(74) Mandataire: Desaix, Anne

(56) Documents cités:
- WO-A-92/01056
- WO-A-93/04202
- WO-A-93/18165
- WO-A-95/00664
- MIRAS I ET AL: "Nucleotide sequence of iagA and iagB genes involved in invasion of HeLa cells by Salmonella enterica subsp. enterica ser. Typhi" RES. MICROBIOLOGY, vol. 146, no. 1, 1 janvier 1995 (1995-01-01), pages 17-20, XP002001767
- CHEVRIER D ET AL: "PCR product quantification by non-radioactive hybridization procedures using oligonucleotide covalently bound to microwells" MOL. CELL. PROBES, vol. 7, 1993, pages 187-97, XP002001768
- CHEVRIER D ET AL: "Rapid detection of Salmonella subspecies I by PCR" FEMS IMMUNOLOGY AND MEDICAL MICROBIOLOGY, vol. 10, février 1995 (1995-02), pages 245-52, XP002001769
- DATABASE MEDLINE [Online] US NATIONAL LIBRARY OF MEDICINE (NLM), BETHESDA, MD, US; April 1999 (1999-04), EUZÉBY J P: "Revised Salmonella nomenclature: designation of Salmonella enterica (ex Kauffmann and Edwards 1952) Le Minor and Popoff 1987 sp. nov., nom. rev. as the neotype species of the genus Salmonella Lignieres 1900 (approved lists 1980), rejection of the name Salmonella choleraesuis (Smith 1894) Weldin 1927" Database accession no. NLM10319519 & INTERNATIONAL JOURNAL OF SYSTEMATIC BACTERIOLOGY APR 1999 LNKD- PUBMED:10319519, vol. 49 Pt 2, April 1999 (1999-04), pages 927-930, ISSN: 0020-7713
- DATABASE MEDLINE [Online] US NATIONAL LIBRARY OF MEDICINE (NLM), BETHESDA, MD, US; July 2000 (2000-07), BRENNER F W ET AL: "Salmonella nomenclature." Database accession no. NLM10878026 & JOURNAL OF CLINICAL MICROBIOLOGY JUL 2000 LNKD- PUBMED:10878026, vol. 38, no. 7, July 2000 (2000-07), pages 2465-2467, ISSN: 0095-1137
- DATABASE MEDLINE [Online] US NATIONAL LIBRARY OF MEDICINE (NLM), BETHESDA, MD, US; January 2005 (2005-01), TINDALL B J ET AL: "Nomenclature and taxonomy of the genus Salmonella." Database accession no. NLM15653930 & INTERNATIONAL JOURNAL OF SYSTEMATIC AND EVOLUTIONARY MICROBIOLOGY JAN 2005 LNKD- PUBMED:15653930, vol. 55, no. Pt 1, January 2005 (2005-01), pages 521-524, ISSN: 1466-5026

### SEQUENCE LISTING

(1) GENERAL INFORMATION:
   (i) APPLICANT: INSTITUT PASTEUR
      INSTITUT NATIONAL DE LA SANTE ET DE LA RECHERCHE MEDICALE
   (ii) TITLE OF INVENTION: OLIGONUCLEOTIDES FOR THE DETECTION OF
      SALMONELLA
   (iii) NUMBER OF SEQUENCES: 29
   (iv) CORRESPONDENCE ADDRESS:
      (A) ADDRESSEE: ERNEST GUTMANN - YVES PLASSERAUD S.A.
      (B) STREET: 3 rue Chauveau-Lagarde
      (C) CITY: PARIS
      (D) STATE:
      (E) COUNTRY: FRANCE
      (F) ZIP: 75008
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: xxxxxxxxxxxx
      (B) FILING DATE: 15-JAN-1996
      (C) CLASSIFICATION:
   (vii) PRIOR APPLICATION DATA:
      (A) APPLICATION NUMBER: FR 9500410
      (B) FILING DATE: 16-JAN-1995
   (vii) PRIOR APPLICATION DATA:
      (A) APPLICATION NUMBER: 96 400 098.8
      (B) FILING DATE: 15-JAN-1996

(2) INFORMATION FOR SEQ ID NO:1:
   (i) SEQUENCE CHARACTERISTICS:
      (A) LENGTH: 2406 base pairs
      (B) TYPE: nucleic acid
      (C) STRANDEDNESS: double
      (D) TOPOLOGY: linear
   (ii) MOLECULE TYPE: DNA (genomic)

   (vi) ORIGINAL SOURCE:
      (A) ORGANISM: SALMONELLA SER. TYPHI
   (ix) FEATURE:
      (A) NAME/KEY: CDS
      (B) LOCATION: 97..1755
      (D) OTHER INFORMATION: /product= "iagA"
   (ix) FEATURE:
      (A) NAME/KEY: CDS
      (B) LOCATION: 1776..2255
      (D) OTHER INFORMATION: /product= "iagB"
   (xi) SEQUENCE DESCRIPTION: SEQ ID NO:1:
   GTACTAGCAG CAGAATTACT GAAACAGTAG ATTCTATCCT AACGACTTGT ATTAGCTATT       60
   ATAACTTTTC ACCCTGTAAG AGAATACACT ATTATC ATG CCA CAT TTT AAT CCT       114
                                           Met Pro His Phe Asn Pro
                                            1               5
   GTT CCT GTA TCG AAT AAA AAA TTC GTC TTT GAT GAT TTC ATA CTC AAC       162
   Val Pro Val Ser Asn Lys Lys Phe Val Phe Asp Asp Phe Ile Leu Asn
               10                  15                  20
   ATG GAC GGC TCC CTC GTA CGC TCA GAA AAG AAA GTC AAT ATT CCG CCA       210
   Met Asp Gly Ser Leu Val Arg Ser Glu Lys Lys Val Asn Ile Pro Pro
           25                  30                  35
   AAA GAA TAT GCC GTT CTG GTC ATC CTG CTC GAA GCC GCC GGC AAG ATT       258
   Lys Glu Tyr Ala Val Leu Val Ile Leu Leu Glu Ala Ala Gly Lys Ile
       40                  45                  50
   GTG AGT AAA AAC ACC TTA TTG GAC CAA GTA TGG GGC GAC GCG GAA GTT       306
   Val Ser Lys Asn Thr Leu Leu Asp Gln Val Trp Gly Asp Ala Glu Val
   55                  60                  65                  70
   AAC GAA GAA TCT CTT ACC CGC TGT ATC TAT GCC TTA CGA CGT ATT CTG       354
   Asn Glu Glu Ser Leu Thr Arg Cys Ile Tyr Ala Leu Arg Arg Ile Leu
                   75                  80                  85
   TCG GAA GAT AAA GAG CAT CGT TAC ATT GAA ACA CTG TAC GGA CAG GGT       402
   Ser Glu Asp Lys Glu His Arg Tyr Ile Glu Thr Leu Tyr Gly Gln Gly
               90                  95                  100
   TAT CGG TTT AAT CGT CCG GTC GTA GTG GTG TCT CCG CCA GCG CCG CAA       450
   Tyr Arg Phe Asn Arg Pro Val Val Val Val Ser Pro Pro Ala Pro Gln
           105                 110                 115
   CCT ACG ACT CAT ACA TTG GCG ATA CTT CCT TTT CAG ATG CAG GAT CAG       498
   Pro Thr Thr His Thr Leu Ala Ile Leu Pro Phe Gln Met Gln Asp Gln
       120                 125                 130
   GTT CAA TCC GAG AGT CTG CAT TAC TCT ATC GTG AAG GGA TTA TCG CAG       546
   Val Gln Ser Glu Ser Leu His Tyr Ser Ile Val Lys Gly Leu Ser Gln
   135                 140                 145                 150
   TAT GCG CCC TTT GGC CTG AGC GTG CTG CCG GTG ACC ATT ACG AAG AAC       594
   Tyr Ala Pro Phe Gly Leu Ser Val Leu Pro Val Thr Ile Thr Lys Asn
                   155                 160                 165
   TGC CGC AGT GTT AAG GAT ATT CTT GAG CTC ATG GAT CAA TTA CGC CCC       642
   Cys Arg Ser Val Lys Asp Ile Leu Glu Leu Met Asp Gln Leu Arg Pro
               170                 175                 180
   GAT TAT TAT ATC TCC GGG CAG ATG ATA CCC GAT GGT AAT GAT AAT ATT       690
   Asp Tyr Tyr Ile Ser Gly Gln Met Ile Pro Asp Gly Asn Asp Asn Ile
           185                 190                 195
   GTA CAG ATC GAG ATA GTT CGG GTT AAA GGT TAT CAC CTG CTG CAC CAG 738
   Val Gln Ile Glu Ile Val Arg Val Lys Gly Tyr His Leu Leu His Gln
   200 205 210
   GAA AGC ATT AAG TTG ATA GAA CAC CAA CCC GCT TCT CTC TTG CAA AAC 786
   Glu Ser Ile Lys Leu Ile Glu His Gln Pro Ala Ser Leu Leu Gln Asn
   215 220 225 230
   AAA ATT GCG AAT CTT TTG CTC AGA TGT ATT CCC GGA CTT CGC TGG GAC 834
   Lys Ile Ala Asn Leu Leu Leu Arg Cys Ile Pro Gly Leu Arg Trp Asp
   235 240 245
   ACA AAG CAA ATT AGC GAG CTA AAT TCG ATT GAC AGT ACC ATG GTC TAC 882
   Thr Lys Gln Ile Ser Glu Leu Asn Ser Ile Asp Ser Thr Met Val Tyr
   250 255 260
   TTA CGC GGT AAG CAT GAG TTA AAT CAA TAC ACC CCC TAT AGC TTA CAG 930
   Leu Arg Gly Lys His Glu Leu Asn Gln Tyr Thr Pro Tyr Ser Leu Gln
   265 270 275
   CAA GCG CTT AAA TTG CTG ACT CAA TGC GTT AAT ATG TCG CCA AAC AGC 978
   Gln Ala Leu Lys Leu Leu Thr Gln Cys Val Asn Met Ser Pro Asn Ser
   280 285 290
   ATT GCG CCT TAC TGT GCG CTG GCA GAA TGC TAC CTC AGC ATG GCG CAA 1026
   Ile Ala Pro Tyr Cys Ala Leu Ala Glu Cys Tyr Leu Ser Met Ala Gln
   295 300 305 310
   ATG GGG ATT TTT GAT AAA CAA AAC GCA ATG ATC AAA GCT AAA GAA CAT 1074
   Met Gly Ile Phe Asp Lys Gln Asn Ala Met Ile Lys Ala Lys Glu His
   315 320 325
   GCT ATT AAG GCG ACA GAG CTG GAC CAC AAT AAT CCA CAA GCT TTA GGA 1122
   Ala Ile Lys Ala Thr Glu Leu Asp His Asn Asn Pro Gln Ala Leu Gly
   330 335 340
   TTA CTG GGG CTA ATT AAT ACG ATT CAC TCA GAA TAC ATC GTC GGG AGT 1170
   Leu Leu Gly Leu Ile Asn Thr Ile His Ser Glu Tyr Ile Val Gly Ser
   345 350 355
   TTA CTA TTC AAA CAA GCT AAC TTA CTT TCG CCC ATT TCT GCA GAT ATT 1218
   Leu Leu Phe Lys Gln Ala Asn Leu Leu Ser Pro Ile Ser Ala Asp Ile
   360 365 370
   AAA TAT TAT TAT GGC TGG AAT CTT TTC ATG GCT GGT CAG TTG GAG GAG 1266
   Lys Tyr Tyr Tyr Gly Trp Asn Leu Phe Met Ala Gly Gln Leu Glu Glu
   375 380 385 390
   GCC TTA CAA ACG ATT AAC GAG TGT TTA AAA TTG GAC CCA ACG CGC GCA 1314
   Ala Leu Gln Thr Ile Asn Glu Cys Leu Lys Leu Asp Pro Thr Arg Ala
   395 400 405
   GCC GCA GGG ATC ACT AAG CTG TGG ATT ACC TAT TAT CAT ACC GGT ATT 1362
   Ala Ala Gly Ile Thr Lys Leu Trp Ile Thr Tyr Tyr His Thr Gly Ile
   410 415 420
   GAT GAT GCT ATA CGT TTA GGC GAT GAA TTA CGC TCA CAA CAC CTG CAG 1410
   Asp Asp Ala Ile Arg Leu Gly Asp Glu Leu Arg Ser Gln His Leu Gln
   425 430 435
   GAT AAT CCA ATA TTA TTA AGT ATG CAG GTT ATG TTT CTT TCG CTT AAA        1458
   Asp Asn Pro Ile Leu Leu Ser Met Gln Val Met Phe Leu Ser Leu Lys
       440                 445                 450
   GGT AAA CAT GAA CTG GCA CGA AAA TTA ACT AAA GAA ATA TCC ACG CAG        1506
   Gly Lys His Glu Leu Ala Arg Lys Leu Thr Lys Glu Ile Ser Thr Gln
   455                 460                 465                 470
   GAA ATA ACA GGA CTT ATT GCT GTT AAT CTT CTT TAC GCT GAA TAT TGT        1554
   Glu Ile Thr Gly Leu Ile Ala Val Asn Leu Leu Tyr Ala Glu Tyr Cys
                   475                 480                 485
   CAG AAT AGT GAG CGT GCC TTA CCG ACG ATA AGA GAA TTT CTG GAA AGT        1602
   Gln Asn Ser Glu Arg Ala Leu Pro Thr Ile Arg Glu Phe Leu Glu Ser
               490                 495                 500
   GAA CAG CGT ATA GAT AAT AAT CCG GGA TTA TTA CCG TTA GTG CTG GTT        1650
   Glu Gln Arg Ile Asp Asn Asn Pro Gly Leu Leu Pro Leu Val Leu Val
           505                 510                 515
   GCC CAC GGC GAA GCT ATT GCC GAG AAA ATG TGG AAT AAA TTT AAA AAC        1698
   Ala His Gly Glu Ala Ile Ala Glu Lys Met Trp Asn Lys Phe Lys Asn
       520                 525                 530
   GAA GAC AAT ATT TGG TTC AAA AGA TGG AAA CAG GAT CCC CGC TTG ATT        1746
   Glu Asp Asn Ile Trp Phe Lys Arg Trp Lys Gln Asp Pro Arg Leu Ile
   535                 540                 545                 550
   AAA TTA CGG TAAAATCTGA GAGAGGAGAT ATG CAT TAT TTT TTT ATC ATC          1796
   Lys Leu Arg                       Met His Tyr Phe Phe Ile Ile
                                       1               5
   GTA ATC TGG TTG CTT AGC ATA AAT ACG GCA TGG GCT GAT TGC TGG CTT        1844
   Val Ile Trp Leu Leu Ser Ile Asn Thr Ala Trp Ala Asp Cys Trp Leu
           10                  15                  20
   CAG GCT GAA AAA ATG TTC AAT ATT GAA TCC GAA CTA CTT TAC GCT ATC        1892
   Gln Ala Glu Lys Met Phe Asn Ile Glu Ser Glu Leu Leu Tyr Ala Ile
       25                  30                  35
   GCC CAG CAG GAA TCG GCG ATG AAA CCT GGC GCC ATT GGT CAT AAC CGA        1940
   Ala Gln Gln Glu Ser Ala Met Lys Pro Gly Ala Ile Gly His Asn Arg
   40                  45                  50                  55
   GAT GGT TCA ACC GAT CTT GGC CTG ATG CAA ATT AAC AGC TTC CAT ATG        1988
   Asp Gly Ser Thr Asp Leu Gly Leu Met Gln Ile Asn Ser Phe His Met
                   60                  65                  70
   AAA AGG CTG AAA AAA ATG GGG ATT AGT GAA AAA CAG TTG TTA CAG GAT        2036
   Lys Arg Leu Lys Lys Met Gly Ile Ser Glu Lys Gln Leu Leu Gln Asp
               75                  80                  85
   CCC TCG ATT TCT GTC ATT GTG GGC GCA TCC ATT TTA TCA GAT ATG ATG        2084
   Pro Ser Ile Ser Val Ile Val Gly Ala Ser Ile Leu Ser Asp Met Met
           90                  95                  100
   AAA ATC TAC GGT TTT AGC TGG GAG GCC GTT GGC GCT TAT AAT GCC GGG        2132
   Lys Ile Tyr Gly Phe Ser Trp Glu Ala Val Gly Ala Tyr Asn Ala Gly
       105                 110                 115
   ACG TCG CCG AAA CGA TCG GAT ATA AGG AAA CGT TAT GCT AAA AAA ATT       2180
   Thr Ser Pro Lys Arg Ser Asp Ile Arg Lys Arg Tyr Ala Lys Lys Ile
   120                 125                 130                 135
   TGG GAG AAT TAC AGA AAA TTA AAA GAG ATG TCA GCA GAA GAG AAA AAC       2228
   Trp Glu Asn Tyr Arg Lys Leu Lys Glu Met Ser Ala Glu Glu Lys Asn
                   140                 145                 150
   AAA AGA CTT TCT ATC GCG GTA AAC AAA TAATTATACA GGAATAGCTT             2275
   Lys Arg Leu Ser Ile Ala Val Asn Lys
               155                 160
   ACTTTCAGAT AATTCTAAAA GTAAGCTATG TTTTTATCAG CTTGCCGTCG TCATAAGCAA     2335
   CTGGCGCTTG CATTGCTTTT AGTTGTACAA ACTGTGAGGC GTCTTCCAGC ATTCTATTGT     2395
   TCCGTGAATT C                                                          2406

(2) INFORMATION FOR SEQ ID NO:2:
   (i) SEQUENCE CHARACTERISTICS:
      (A) LENGTH: 553 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Pro His Phe Asn Pro Val Pro Val Ser Asn Lys Lys Phe Val Phe
     1               5                  10                  15
   Asp Asp Phe Ile Leu Asn Met Asp Gly Ser Leu Val Arg Ser Glu Lys
                20                  25                  30
   Lys Val Asn Ile Pro Pro Lys Glu Tyr Ala Val Leu Val Ile Leu Leu
            35                  40                  45
   Glu Ala Ala Gly Lys Ile Val Ser Lys Asn Thr Leu Leu Asp Gln Val
        50                  55                  60
   Trp Gly Asp Ala Glu Val Asn Glu Glu Ser Leu Thr Arg Cys Ile Tyr
    65                  70                  75                  80
   Ala Leu Arg Arg Ile Leu Ser Glu Asp Lys Glu His Arg Tyr Ile Glu
                    85                  90                  95
   Thr Leu Tyr Gly Gln Gly Tyr Arg Phe Asn Arg Pro Val Val Val Val
               100                 105                 110
   Ser Pro Pro Ala Pro Gln Pro Thr Thr His Thr Leu Ala Ile Leu Pro
           115                 120                 125
   Phe Gln Met Gln Asp Gln Val Gln Ser Glu Ser Leu His Tyr Ser Ile
       130                 135                 140
   Val Lys Gly Leu Ser Gln Tyr Ala Pro Phe Gly Leu Ser Val Leu Pro
   145                 150                 155                 160
   Val Thr Ile Thr Lys Asn Cys Arg Ser Val Lys Asp Ile Leu Glu Leu
                   165                 170                 175
   Met Asp Gln Leu Arg Pro Asp Tyr Tyr Ile Ser Gly Gln Met Ile Pro
               180                 185                 190
   Asp Gly Asn Asp Asn Ile Val Gln Ile Glu Ile Val Arg Val Lys Gly
           195                 200                 205
   Tyr His Leu Leu His Gln Glu Ser Ile Lys Leu Ile Glu His Gln Pro
       210                 215                 220
   Ala Ser Leu Leu Gln Asn Lys Ile Ala Asn Leu Leu Leu Arg Cys Ile
   225                 230                 235                 240
   Pro Gly Leu Arg Trp Asp Thr Lys Gln Ile Ser Glu Leu Asn Ser Ile
                   245                 250                 255
   Asp Ser Thr Met Val Tyr Leu Arg Gly Lys His Glu Leu Asn Gln Tyr
               260                 265                 270
   Thr Pro Tyr Ser Leu Gln Gln Ala Leu Lys Leu Leu Thr Gln Cys Val
           275                 280                 285
   Asn Met Ser Pro Asn Ser Ile Ala Pro Tyr Cys Ala Leu Ala Glu Cys
       290                 295                 300
   Tyr Leu Ser Met Ala Gln Met Gly Ile Phe Asp Lys Gln Asn Ala Met
   305                 310                 315                 320
   Ile Lys Ala Lys Glu His Ala Ile Lys Ala Thr Glu Leu Asp His Asn
                   325                 330                 335
   Asn Pro Gln Ala Leu Gly Leu Leu Gly Leu Ile Asn Thr Ile His Ser
               340                 345                 350
   Glu Tyr Ile Val Gly Ser Leu Leu Phe Lys Gln Ala Asn Leu Leu Ser
           355                 360                 365
   Pro Ile Ser Ala Asp Ile Lys Tyr Tyr Tyr Gly Trp Asn Leu Phe Met
       370                 375                 380
   Ala Gly Gln Leu Glu Glu Ala Leu Gln Thr Ile Asn Glu Cys Leu Lys
   385                 390                 395                 400
   Leu Asp Pro Thr Arg Ala Ala Ala Gly Ile Thr Lys Leu Trp Ile Thr
                   405                 410                 415
   Tyr Tyr His Thr Gly Ile Asp Asp Ala Ile Arg Leu Gly Asp Glu Leu
               420                 425                 430
   Arg Ser Gln His Leu Gln Asp Asn Pro Ile Leu Leu Ser Met Gln Val
           435                 440                 445
   Met Phe Leu Ser Leu Lys Gly Lys His Glu Leu Ala Arg Lys Leu Thr
       450                 455                 460
   Lys Glu Ile Ser Thr Gln Glu Ile Thr Gly Leu Ile Ala Val Asn Leu
   465                 470                 475                 480
   Leu Tyr Ala Glu Tyr Cys Gln Asn Ser Glu Arg Ala Leu Pro Thr Ile
                   485                 490                 495
   Arg Glu Phe Leu Glu Ser Glu Gln Arg Ile Asp Asn Asn Pro Gly Leu
               500                 505                 510
   Leu Pro Leu Val Leu Val Ala His Gly Glu Ala Ile Ala Glu Lys Met
           515                 520                 525
   Trp Asn Lys Phe Lys Asn Glu Asp Asn Ile Trp Phe Lys Arg Trp Lys
       530                 535                 540
   Gln Asp Pro Arg Leu Ile Lys Leu Arg
   545                 550
(2) INFORMATION FOR SEQ ID NO:3:
   (i) SEQUENCE CHARACTERISTICS:
      (A) LENGTH: 160 amino acids
      (B) TYPE: amino acid
      (D) TOPOLOGY: linear
   (ii) MOLECULE TYPE: protein
   (xi) SEQUENCE DESCRIPTION: SEQ ID NO:3:
   Met His Tyr Phe Phe Ile Ile Val Ile Trp Leu Leu Ser Ile Asn Thr
     1               5                  10                  15
   Ala Trp Ala Asp Cys Trp Leu Gln Ala Glu Lys Met Phe Asn Ile Glu
                20                  25                  30
   Ser Glu Leu Leu Tyr Ala Ile Ala Gln Gln Glu Ser Ala Met Lys Pro
            35                  40                  45
   Gly Ala Ile Gly His Asn Arg Asp Gly Ser Thr Asp Leu Gly Leu Met
        50                  55                  60
   Gln Ile Asn Ser Phe His Met Lys Arg Leu Lys Lys Met Gly Ile Ser
    65                  70                  75                  80
   Glu Lys Gln Leu Leu Gln Asp Pro Ser Ile Ser Val Ile Val Gly Ala
                    85                  90                  95
   Ser Ile Leu Ser Asp Met Met Lys Ile Tyr Gly Phe Ser Trp Glu Ala
               100                 105                 110
   Val Gly Ala Tyr Asn Ala Gly Thr Ser Pro Lys Arg Ser Asp Ile Arg
           115                 120                 125
   Lys Arg Tyr Ala Lys Lys Ile Trp Glu Asn Tyr Arg Lys Leu Lys Glu
       130                 135                 140
   Met Ser Ala Glu Glu Lys Asn Lys Arg Leu Ser Ile Ala Val Asn Lys
   145                 150                 155                 160
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:4:
      TATTAAGTAT GCAGGTTATG 20
(2) INFORMATION FOR SEQ ID NO:5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:5: AGAGAATTTC TGCAAAGTGA A 21
(2) INFORMATION FOR SEQ ID NO:6:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:6:
      ATATCCACGC AGGAAATAAC AGGACTT 27
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:7:
      GAGCGTGCCT TACCGACGAT A 21
(2) INFORMATION FOR SEQ ID NO:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:8:
      GCAGGGATCA CTAAGCTGTG 20
(2) INFORMATION FOR SEQ ID NO:9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:9:
      CGTGGGCAAC CAGCACTAAC G 21
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:10:
      CGGGTTAAAG GTTATCACCT 20

(2) INFORMATION FOR SEQ ID NO:11:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:11:
      AGCATGGCGC AAATGGG 17

(2) INFORMATION FOR SEQ ID NO:12:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:12:
      GCACCAGGAA AGCATTAAGT TGATAGAACA C 31
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:13:
      CTTCGCTGGG ACACAAAGCA 20
(2) INFORMATION FOR SEQ ID NO:14:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:14:
      TAATGCTTTC CTGGTGC 17
(2) INFORMATION FOR SEQ ID NO:15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:15:
      TACGGCATGG GCTGATTGCT 20
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:16:
      TTACGCTATC GCCCAGCAGC AGGA 24
(2) INFORMATION FOR SEQ ID NO:17:
   (i) SEQUENCE CHARACTERISTICS:
      (A) LENGTH: 29 base pairs
      (8) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:17:
      TGGTCATAAC CGAGATGGTT CAAACGATC 29
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:18:
      ACAGTTGTTA CAGGATCCCT 20
(2) INFORMATION FOR SEQ ID NO:19:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:19:
      CCGGGCAGAT GATACCC 17
(2) INFORMATION FOR SEQ ID NO:20:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:20:
   TATTATCATA CCGGTATTGA TGATGCTATA CGTTTAGGCG ATGAATTACG CTCACAACAC      60
   CTGCAGGATA ATCCAATATT ATTAAGTATG CAGGTTATGT TTCTTTCGCT TAAAGGTAAA     120
   CATGAACTGG CACGAAAATT AACTAAAGAA ATATCCACGC AGGAAATAAC AGGACTTATT     180
   GCTGTTAATC TTCTTTACGC TGAATATTGT CAGAATAGTG AGCGTGCCTT ACCGACGATA     240
   AGAGAATTTC TGGAAAGTGA ACAGCGTATA GATAATAATC CGGGATTATT ACCGTTAGTG     300

(2) INFORMATION FOR SEQ ID NO:21:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:21:
   TATTATCATA CTGGTATTGA TGATGCTATA CGTTTAGGCG ATGAATTACG CTCACAACAC      60
   CTGCAGGATA ATCCAATATT ATTAAGTATG CAGGTTATGT TTCTTTCTCT TAAAGGTAAA     120
   CATGAACTGG CACGAAAATT ATCTAAAGAA ATATCCACGC AGGAAATAAC AGGGCTTATT     180
   GCTGTTAATC TTCTTTATGC TGAATACTGT CAGAATAGTG AGCGTGCCTT ACCGACGATA     240
   AGAGAATTTC TGGAAAGTGA ACAGCGTATA GATAATAATC CGGGATTATT ACCGTTAGTG     300

(2) INFORMATION FOR SEQ ID NO:22:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:22:
   TATTATCATA CCGGCATTGA TGATGCCATA CGTTTAGGAG ATGAACTACG CTCACAGCAC      60
   CTGCAGGATA ATCCCATTTT ATTAAGTATG CAGGTTATGT TTCTTTCGCT TAAAGGTAAA     120
   CATGAGCTGG CACGAAAATT AACTAAAGAG ACATCCCCGC ATGAGATAAC AGGGCTTATT     180
   GCTATTAATC TTCTTTATGC TGAATACTGT CAGAATAGTG AGCGAGCCTT ACCGAGGATA     240
   AGAGAATATC TGGCAAGTGA ACGGAGTATT GATAATAATC CTGGACTATT ACCGTTAGTG     300

(2) INFORMATION FOR SEQ ID NO:23:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:23:
   TATTATCATG CCGGCATTGA TGATGCTATA CGTTTAGGAG ATGAATTACG TTCACAACAT 60
   CTGCAGGATA ATCCAATACT ATTAAGTATG CAGGTTATGT TTCTTTCGCT TAAAGGTAAA 120
   CATGAACTGG CACGAAAATT AGCTAAAGAA ATATCCAAGC ATGAAATAAC AGGGCTTATT 180
   GCTGTTAATC TTCTGTATGC TGAATACTGT CAGAATAGCG AGCGTGCATT ACCGAGGATA 240
   AGAGAGTTTC TGGAAAGTGA ACAGAATACT GATAATAATC CCGGGCTATT ACCGTTAGTG 300

(2) INFORMATION FOR SEQ ID NO:24:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:24:
   TACTATCATA CTGGCCTTGA TGATGCTATA CGTTTAGGAG ATGAATTACG TTCGCAACAT 60
   TTGCAGGATA ATCCAATATT ATTAAGTATG CAGGTTATGT TTCTTTCGCT TAAAGGTAAA 120
   CATGAACTGG CACGAAAATT AACTAAAGAA ATATCCACAC ATGAAATAAC AGGGCTTATT 180
   GCTGTTAATC TTCTTTATGC TGAATACTGT CAGAATAGTG AGCGTGCCTT AGCGACGATA 240
   AGAGAATTTC TGGAAAGTGA ACAGAGTGTT GATAATAACC CAGGGTTATT ACCGTTAGTG 300

(2) INFORMATION FOR SEQ ID NO:25:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear

   (ii) MOLECULE TYPE: DNA (genomic)
   (xi) SEQUENCE DESCRIPTION: SEQ ID NO:25:
   TATTATCAGA CTGGCATTGA TGATGCTATA CGTTTAGGCG ATGAATTACG CTCACAATAT 60
   CTGCAAGATA ATCCAATATT ATTAAGTATG CAGCTTATGT TTCTTTCGCT TAAAGGTAAA 120
   CATGAGTTGG CACGAAAATT AGCTAAAGAA ATATCCACAC ACGAAGTAAC AGGGCTTATT 180
   GCTGTTAATC TTCTTTATGC TGAATACTGT CAGAATAGCG AGCGTGCTTT ACCGGCGATA 240
   AGAGAATTTC TGGAAAGTGA ACAGAATATA GATAATAATC CGGGGCTATT ACCGTTAGTG 300

(2) INFORMATION FOR SEQ ID NO:26:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:26:
   TACTATCATA CAGGCCTTGA TGATGCTATA CGTTTAGGCG ATGAATTACG TACACAACAT    60
   TTGCAAGATA ATCCTATATT ATTAAGTATG CAAGTTATGT TTCTTTCGCT TAAAGGTAAA   120
   CATGAACTGG CACGGCTATT AGCTAAAGAA ATATCCTCAC ATGAAATAAC AGGGCTTATT   180
   GCTATTAATC TTCTTTATGC TGAATATTGT CAGAATAGTG AGCGCGCCTT ACCGGCGATA   240
   AAAGAATTTC TGGAAAGTGA ACAAAATATT GACAATAACC CTGGGCTATT ACCGTTAGTG   300

(2) INFORMATION FOR SEQ ID NO:27:
   (i).SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:27:
   TATTATCATA CCGGCATTGA TGATGCTATA CGGTTAGGAG ATGAATTACG TTCACAACAC    60
   TTGCAGGATA ATCCAATATT ATTAAGTATG CAGGTTATGT TTCTTTCGCT TAAAGGTAAA   120
   CATGAACTGG CACGAAAATT AACTAAAGAA ATATCCAGAC ATGAAATAAC AGGGCTTATT   180
   GCTGTTAATC TTCTTTATGC TGAATACTGT CAGAATAGTG AGCGTGCCTT ATCGAGGATA   240
   AGAGAATTTC TGGAAAGTGA ACAGAGTATT GATAATAATC CAGGACTATT ACCGTTAGTG   300

(2) INFORMATION FOR SEQ ID NO:28:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:28:
      CCCGAACTAT CTCGATCTGT ACAATATTAT CATT 34
(2) INFORMATION FOR SEQ ID NO:29:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid

   (xi) SEQUENCE DESCRIPTION: SEQ ID NO:29:
      GCAGGTGATT ACCTTTAA 18

## Description

Le genre Salmonella contient deux espèces, Salmonella enterica, espèce divisée en six sous-espèces, sur la base de caractères biochimiques et d'homologies au niveau de l'ADN, et Salmonella bongori. Le genre est subdivisé en plus de 2000 sérovariétés définies à l'aide d'antigènes somatiques et flagellaires. Les bactéries du genre Salmonella sont de façon générale pathogènes pour l'animal ou pour l'homme. On sait ainsi que les Salmonella sont parmi les agents responsables des empoisonnements alimentaires les plus courants dans les pays développés ; c'est pourquoi des méthodes de détection rapides et fiables des sous-espèces de Salmonella sont importantes.

Les salmonelles responsables des toxi-infections alimentaires appartiennent majoritairement à la sous-espèce I (encore appelée groupe I) de S. enterica.

Les toxi-infections ne sont toutefois pas les seules pathologies provoquées par des infections par des Salmonella.

Par exemple, Salmonella enterica sous-espèce enterica sérovariété typhi (ci-dessous dénommée Typhi) est l'agent causal de la fièvre typhoïde humaine.

Compte tenu de la nature des infections provoquées par les salmonelles et de la nécessité notamment de rechercher leur présence dans les prélèvements biologiques réalisés sur des patients ou dans les aliments, il apparaît indispensable de disposer de moyens rapides et sensibles pour en détecter leur présence.

Les méthodes standard de culture largement utilisées jusqu'à présent pour la détection des salmonelles nécessitent un temps important et ne sont pas adaptées par exemple pour suivre la contamination de produits alimentaires. Pour surmonter les désavantages de ces méthodes, plusieurs méthodes fondées sur des techniques de biologie moléculaire telles que des tests d'hybridation et des tests de réaction de polymérisation en chaîne, ont d'ores et déjà été proposées. Différentes sondes d'ADN ont été utilisées dans plusieurs protocoles d'hybridation et de PCR pour détecter les sous-espèces de Salmonella dans l'alimentation. Cependant, aucune de ces techniques n'est complètement satisfaisante, puisque les séquences utilisées ne sont pas totalement connues ou pas exclusivement présentes dans le genre Salmonella et ainsi, peuvent conduire à des réactions croisées entre la sonde et des séquences d'ADN d'autres enterobactéries ou peuvent conduire à un grand nombre de faux négatifs ou de faux positifs.

Les inventeurs ont recherché des moyens permettant la détection spécifique et sensible de l'ensemble des salmonelles des espèces S. enterica et/ou S. bongori. Dans cette optique, ils se sont intéressés à la souche Salmonella enterica sous-espèce enterica sérovariété typhi (S. Typhi) et au gène participant à l'invasion de cellules par S. Typhi.

De plus, ils ont défini certaines conditions permettant la détection spécifique de groupes déterminés de Salmonelles, par exemple des bactéries du Groupe I.

On a déjà montré dans l'état antérieur de la technique, que la souche Typhi est capable d'adhérer à des monocouches de cellules HeLa et d'entrer dans ces cellules (Yabuuchi et al, 1986). Cependant, jusqu'à présent, les déterminants génétiques impliqués dans ce processus d'adhésion et d'entrée dans les cellules, n'ont pas été clairement identifiés. Eisinghorst et al (1989) ont cloné un fragment chromosomique de Typhi, qui confère à des bactéries de type Escherichia coli la capacité de pénétrer dans les cellules Henle 407. Récemment, une autre région chromosomique impliquée dans l'invasion des cellules HeLa par la souche Typhi Ty2 a été identifiée et clonée (Popoff et Dion, 1990).

Les inventeurs de la présente demande ont identifié sur un fragment d'ADN de 2,4 kb de S. typhi contenu dans la séquence HindIII de 7,9 kb décrite par Popoff et Dion (1990), des régions susceptibles de participer à l'activité d'invasion de Salmonella enterica sous-espèce enterica sérovariété Typhi dans des cellules, et en particulier dans des cultures cellulaires de type HeLa, ces régions étant susceptibles en outre d'être utilisées dans des réactions pour la réalisation d'un diagnostic généralisé de tous les représentants des espèces S. enterica et/ou S. bongori ou éventuellement dans des conditions de détection particulières, pour le diagnostic spécifique du groupe I de S. enterica.

Une séquence appelée IagA et une séquence appelée IagB ont été identifiées par les inventeurs et caractérisées comme participant à l'invasion cellulaire se manifestant lors d'une infection dûe à Salmonella enterica sous-espèce enterica sérovariété Typhi.

La spécificité de ces séquences au sein de S. Typhi a conduit les inventeurs à proposer leur utilisation pour définir des moyens pour le diagnostic d'une infection par S. typhi, voire pour le diagnostic d'une infection par des Salmonella des espèce S. enterica et/ou S. bongori ou dans certains cas pour la mise en évidence de S. enterica de groupes spécifiques.

Ces moyens utilisables pour le diagnostic d'une infection par Salmonella enterica et/ou Salmonella bongori comprennent des oligonucléotides susceptibles d'être mis en oeuvre dans des réactions d'amplification de séquences de nucléotides, par exemple des réactions de polymérisation en chaîne. La demande décrit aussi des sondes pour la détection d'acides nucléiques de S. enterica ou d'une sous-espèce spécifique de S. enterica et/ou de S. bongori, ces acides nucléiques étant le cas échéant des fragments amplifiés.

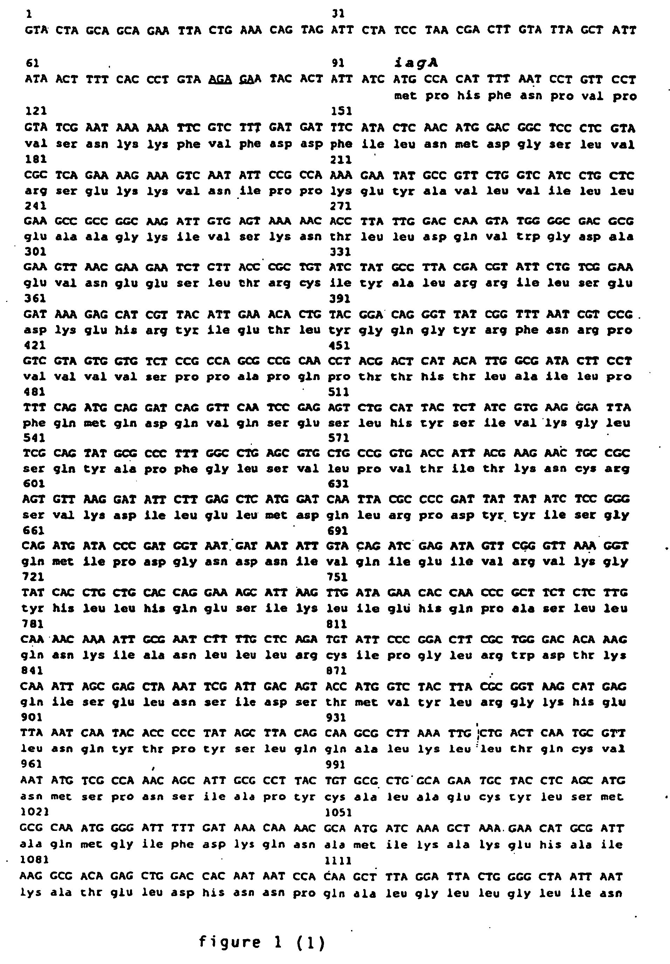

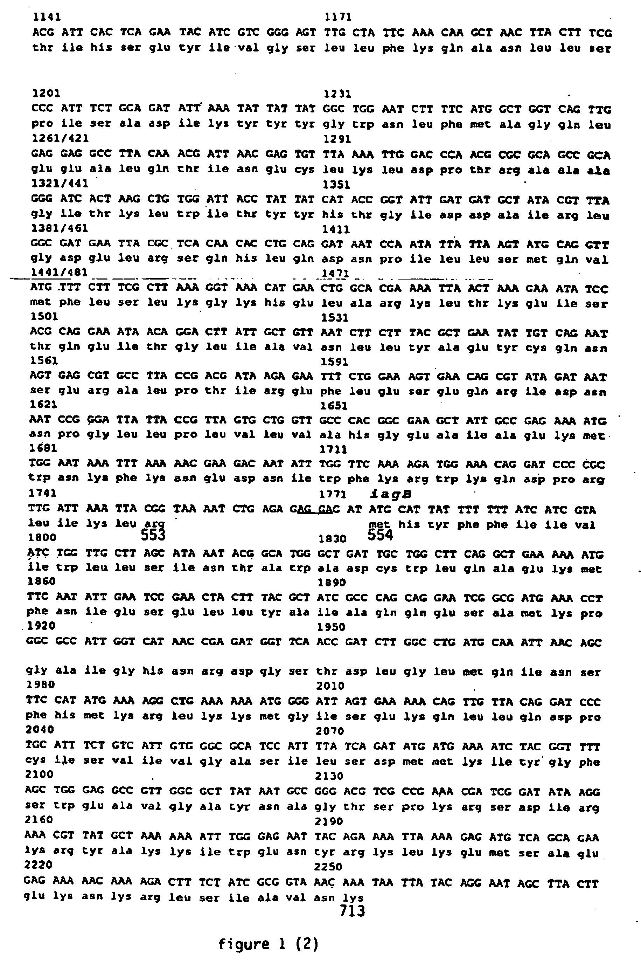

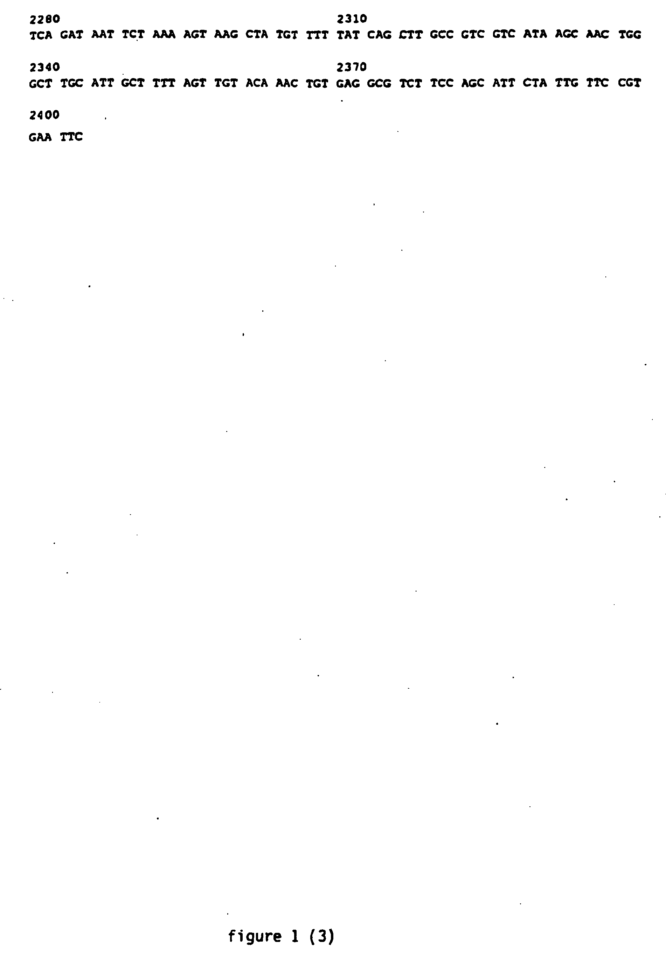

L'invention a également pour objet un kit et une méthode de détection de la présence de Salmonella enterica et de Salmonella bongori dans des échantillons biologiques et par exemple, dans des produits alimentaires ou dans tout échantillon faisant l'objet d'un diagnostic clinique. D'autres méthodes et kits de détection sont spécifiques des souches du groupe I de S. enterica. Ces autres méthodes permettent au contraire de rechercher la présence de bactéries S. enterica ou S. bongori du genre Salmonella. Le genre Salmonella comporte ainsi six sous-espèces ou groupes I, II, III, IV, V ou VI. Les sous-espèces I, II, III, IV et VI appartiennent à l'espèce S. enterica et la sous-espèce V appartient à l'espèce S. bongori. La demande décrit aussi les séquences de nucléotides, participant à l'invasion de cellules par Salmonella enterica sous-espèce enterica sérovariété Typhi, **caractérisées en ce qu'**il s'agit de l'une des séquences iagA ou iagB respectivement comprises entre les nucléotides 97 et 1755 de la séquence représentée à la figure 1 (IagA) et entre les nucléotides 1776 et 2255 de la séquence représentée à la figure 1 (IagB).

La demande décrit aussi des séquences de nucléotides modifiées par rapport à iagA ou iagB mais présentant néanmoins les mêmes propriétés s'agissant de l'invasion des cellules, ou hybridant dans des conditions stringentes avec l'une des susdites séquences.

La présente demande décrit également des protéines IagA et IagB répondant aux séquences présentées à la figure 1 ou des variantes de ces séquences obtenues par mutation, délétion ou addition d'acides aminés dès lors que la séquence ainsi obtenue est reconnue par des anticorps dirigés contre l'une des susdites séquences IagA ou IagB.

De façon générale, l'invention a pour objet toute séquence d'acides aminés codée par les gènes iagA et iagB représentés à la figure 1.

L'invention concerne par ailleurs tout fragment de l'une de ces séquences, en particulier tout fragment sous forme purifiée, suffisant pour conserver à S. typhi ses propriétés d'adhésion et d'infection des cellules, et en particulier des cellules HeLa en culture.

Le procédé d'infection des cellules HeLa en culture est le procédé standard qui a notamment été décrit dans la demande de brevet internationale publiée sous le numéro WO 92/01056.

Selon un autre aspect, l'invention concerne des moyens pour la détection de la présence de S. enterica et/ou S. bongori et le cas échéant, pour la quantification de S. enterica et/ou S. bongori dans des échantillons biologiques.

Par échantillon biologique, on entend tout échantillon prélevé pour la réalisation d'analyses in vitro chez l'animal ou chez l'homme ou prélevé à partir de produits alimentaires quelle qu'en soit la nature ou à partir de tout milieu liquide, solide ou gazeux susceptible de contenir les agents pathogènes recherchés.

L'invention a pour objet dans ce cadre, une séquence de nucléotides, comportant au moins 9 nucléotides, **caractérisée en ce qu'**elle hybride avec l'une des séquences IagA ou IagB présentées ci-dessus.

Les conditions d'hybridation dont il est question précédemment sont définies en fonction de la spécificité recherchée de l'hybridation et des conditions appropriées sont données à titre indicatif dans les exemples de la présente demande.

L'invention utilise des oligonucléotides issus de la partie C-terminale de la séquence iagA représentée à la figure 1.

Des séquences de type oligonucléotides peuvent être sélectionnées pour être utilisées comme amorces soit pour la détection après amplification, de l'ADN génomique ou de l'ADNc de Salmonella de l'espèce S. enterica et/ou de l'espèce S. bongori appartenant aux autres groupes I à VI.

Il peut s'agir notamment de séquences de nucléotides obtenues par synthèse chimique selon les métodes connues de l'homme du métier.

Des oligonucléotides préférés, utilisables pour l'amplification d'acide nucléique caractéristique de bactéries appartenant à l'un des groupes I, II, IIIa, IIIb, IV, V ou VI du genre Salmonella et notamment de l'ADN génomique ou de l'ADNc de S. enterica et/ou de S. bongori sont par exemple les suivants (leur position au sein de la séquence IagA représentée à la figure 1 étant indiquée) :

| | | position |
|---|---|---|
| Iag1: | 5'-TA TTA AGT ATG CAG GTT ATG - 3' | 1424-1443 |
| Iag2: | 5'-AGA GAA TTT CTG GAA AGT GAA- 3 ' | 1585-1605 |
| Iag3: | 5'-ATA TCC ACG CAG GAA ATA ACA GGA CTT -3' Iag4: 5'-GAG CGT GCC TTA CCG ACG ATA-3' | 1495-1521 1564-1584 |
| Iag5: | 5'-GCA GGG ATC ACT AAG CTG TG-3' | 1318-1337 |
| Iag6: | 5'-CGT GGG CAA CCA GCA CTA ACG-3' | 1637-1657. |
| De plus, les oligonucléotides | | |
| Slm1: | 5'-CG GGT TAA AGG TTA TCA CGT-3' | 709-728 |
| Slm2: | 5'-AG CAT GGC GCA AAT GGG-3' | 1014-1031 |
| Slm3: | 5'-GCA CCA GGA AAG CAT TAA GTT GAT AGA ACA C-3' | 732-762 |
| Slm4: | 5'-CTT CGC TGG GAC ACA AAG CA-3' | 823-842 et |
| SS28: | 5'-TAA TGC TTT CCT GGT GC-3' sont divulgués | |

D'autres oligonucléotides susceptibles d'être utilisés comme amorces pour l'amplification de l'ADN ou de l'ADNc du gène iagB de l'ensemble des souches Salmonella des espèces S. enterica et/ou S. bongori ont été définies à partir de la séquence iagB représentée à la figure 1.

La présente demande décrit donc les oligonucléotides répondant aux enchainements suivants :
Iag7: 5'-T ACG GCA TGG GCT GAT TGC T-3'
Lag8: 5'-T TAC GCT ATC GCC CAG CAG CAG GA-3'
Iag9: 5'-T GGT CAT AAC CGA GAT GGT TCA ACC GAT C-3'
Iag10: 5'-A CAG TTG TTA CAG GAT CCC T-3'

Ces oligonucléotides peuvent également être utilisés comme sondes, par exemple pour la détection des produits d'amplification de l'ADN et/ou de l'ADNc de S. enterica et/ou de S. bongori.

Un couple d'amorces préféré pour réaliser l'amplification de l'acide nucléique de S. enterica et/ou de S. bongori, quel que soit le groupe d'appartenance de la bactérie, est par exemple formé des amorces Iag5 (sens) et Iag6 (antisens).

Ce couple d'amorces dirige l'amplification d'un fragment d'acide nucléique de 340 bp.

Un autre couple d'amorces est formé des amorces Slm1 (sens) et Slm2 (antisens). Ces amorces sont susceptibles de s'hybrider avec l'ADN ou l'ADNc de bactéries S. enterica et/ou de S. bongori de l'un des groupes I, II, III, IV, V ou VI.

Des oligonucléotides utilisables comme amorces pour la détection spécifique de Salmonella enterica Groupe I lorsque les conditions de détection après l'amplification de l'ADN ou de l'ADNc sont celles qui sont décrites dans l'exemple I.

De telles amorces sont caractérisées par leur capacité à amplifier des séquences d'acide nucléique des souches de S. enterica ou de S. bongori représentatives des groupes I, II, III, IV, V et VI, mais pour lesquelles les conditions de détection sont celles exposées dans l'exemple I, permettant la seule détection des bactéries du groupe I.

Un couple d'oligonucléotides utilisables à ces fins, en tant qu'amorces spécifiques pour la détection de séquences d'ADN ou d'ADNc de S. enterica du groupe I est par exemple constitué par les séquences suivantes :
SS2 5'-CCGGGCAGATGATACCC-3' et
SS28 '-TAATGCTTTCCTGGTGC-3'.

Les oligonucléotides définis par les inventeurs, permettent d'envisager lue diagnostic de S. enterica et de S. bongori dans des conditions satisfaisantes de sensibilité de rapidité, de facilité et de spécificité.

L'invention également a pour objet un kit pour la détection de S. enterica et de S. bongori par amplification de l'ADN génomique ou complémentaire de S. enterica et/ou de S. bongori, **caractérisé en ce qu**'il comprend :
- des oligonucléotides tels que décrits précédemment, capables d'hybrider dans des conditions stringentes avec l'ADN génomique ou l'ADNc de S. enterica et de S. bongori,
- une sonde pour la détection des fragments amplifiés répondant à l'une des définitions données dans les pages précédentes,
- les réactifs nécessaires à la réalisation de la réaction d'amplification.

La présente demande décrit l'utilisation des oligonucléotides précités, comme amorces pour l'amplification d'une séquence d'ADN ou d'ADNc de Salmonella enterica et/ou de Salmonella bongori, comprise dans l'une des séquences iagA ou iagB telles que décrites dans les pages précédentes ou complémentaires d'une telle séquence, ou encore l'utilisation de ces oligonucléotides comme sonde pour la détection d'une séquence de nucléotides amplifiée.

L'invention a donc en particulier pour objet, l'utilisation d'un couple d'amorces oligonucléotidiques tel que défini à la revendication 1 pour l'amplification d'une séquence d'ADN ou d'ADNc de *Salmonella enterica* et *Salmonella bongori* ou encore l'utilisation de ce couple d'amorces oligonucléotidiques comme sonde pour la détection d'une séquence de nucléotides amplifiée.

Par exemple, les oligonucléotides iag5 et iag6 peuvent être utilisés respectivement comme amorces sens et antisens pour la détection de S. enterica et de S. bongori du groupe I, II, III, IV, V ou VI.
En dehors de l'étendue des revendications, le couple d'amorces Slm1 et Slm2 peut être utilisé pour la détection de bactéries de l'espèce S. enterica et/ou de S. bongori de l'un de ces groupes dans un échantillon biologique.

L'utilisation des oligonucléotides SS2 et SS28 pour la détection spécifique in vitro dans un échantillon biologique de S. enterica du groupe I est divulguée.

La détection est spécifique lorsque les amorces utilisées pour l'amplification des séquences de nucléotides recherchées permettent l'amplification de bactéries S. enterica et de S. bongori appartenant à l'un des autres groupes II, III, IV, V ou VI, mais que les conditions mises en oeuvre ne permettent pas la détection des bactéries de ces mêmes groupes ou d'organismes différents susceptibles d'être présents dans l'échantillon biologique testé.

La demande décrit ainsi un ensemble d'oligonucléotides utilisables pour la détection de bactéries S. enterica et/ou de S. bongori, après amplification de l'ADN génomique ou complémentaire de S. enterica et/ou de S. bongori, **caractérisé en ce qu'**il comprend :
- un couple d'oligonucléotides répondant aux définitions précédemment données, capables d'hybrider dans des conditions stringentes avec l'ADN génomique ou l'ADNc de S. enterica et/ou de S. bongori,
- une sonde répondant aux caractéristiques données ci-dessus.

Un premier ensemble d'oligonucléotides utilisables pour la détection in vitro dans un échantillon biologique, de souches de Salmonella enterica et de S. bongori appartenant à l'un des groupes I, II, III, IV, V ou VI, est **caractérisé en ce qu'**il contient les oligonucléotides suivants :
- la séquence Iag5 (5'-GCA GGG ATC ACT AAG CTG TG-3' et la séquence Iag6 (5'-CGT GGG CAA CCA GCA CTA ACG-3') utilisables en tant qu'amorces pour l'amplification et
- la séquence Iag3 (5'-ATA TCC ACG CAG GAA ATA ACA GGA CTT -3') utilisable comme sonde de révélation et la séquence Iag4 (5'-GAG CGT GCC TTA CCG ACG ATA-3') utilisable comme sonde de capture.

Un autre ensemble d'oligonucléotides utilisables pour la détection spécifique in vitro dans un échantillon biologique, de S. enterica du groupe I, est **caractérisé en ce qu'**il comprend les oligonucléotides suivants :
SS2 (5'-CCGGGCAGATGATACCC-3' et
SS28 ('-TAATGCTTTCCTGGTGC-3').

La présente demande décrit par ailleurs une protéine iagA codée par la séquence de nucléotides iagA représentée à la figure 1 ainsi qu'une protéine iagB codée par la séquence de nucléotides iagB représentée à la figure 1.

Les protéines iagA et iagB ont respectivement les séquences d'acides aminés représentées à la figure 1.

La demande décrit également un procédé pour la détection in vitro dans un échantillon biologique, de séquences de nucléotides Salmonella enterica et/ou de S. bongori, préalablement amplifiées par exemple par PCR **caractérisé en ce qu'**il comprend les étapes de :
- dénaturation de la séquence de S. enterica et/ou de S. bongori amplifiée,
- mise en contact des séquences de nucléotides amplifiées dénaturées de S. enterica et/ou de S. bongori, avec une sonde de capture et une sonde de révélation obtenues à partir des oligonucléotides ci-dessus définis dans des conditions permettant l'hybridation desdites sondes de capture et de révélation avec la susdite séquence de nucléotides amplifiée de S. enterica et/ou de S. bongori, la sonde de capture étant fixée à la surface d'un puits d'une plaque de microtitration et la sonde de révélation étant marquée et libre dans un tampon d'hybridation approprié ;
- incubation du mélange réactionnel, pendant un temps suffisant pour permettre la réaction d'hybridation ;
- lavage pour éliminer les oligonucléotides n'ayant pas réagi ;
- révélation des sondes de révélation ayant hybridé aux séquences de nucléotides amplifiées.

Le procédé de détection précédemment décrit peut être avantageusement **caractérisé en ce que** la détection est réalisée conformément aux étapes suivantes :
- dénaturation d'un volume 10 µl de la séquence amplifiée par addition volume à volume d'une solution 200 mM MaOH, 40mM EDTA,
- préhybridation des microplaques dont la surface des puits lest revêtue de la sonde de capture, dans un tampon d'hybridation approprié,
- libération de la microplaque et remplissage de chacune des cupules avec 200µl de tampon d'hybridation renfermant le fragment amplifié dénaturé et la sonde de révélation marquée à la peroxydase à la concentration de 10ng/µl,
- incubation du mélange pendant une heure à 37°C sous agitation,
- lavage du mélange ayant réagi avec une solution de lavage 10X (100mM Tris, 3M NaCl, 1% Tween 20, pH 7,4),
- détection de l'activité de la peroxydase liée à la sonde par colorimétrie en présence d'un substrat coloré.

La révélation de l'activité de la peroxydase présente sur la sonde de révélation peut être obtenue par mise en oeuvre des étapes suivantes :
- dépôt de 200µl d'une solution 40mM citrate trisodique, 0,03% H₂O 30%, 7,5mg/ml d'orthophenylenediamine (OPD) dans chacun des puits contenant le mélange de réaction,
- incubation de la microplaque pendant 30min à l'obscurité et à 37°C,
- bloquage de la réaction par addition de 50µl/puits d'une solution 4N H₂SO₄,
- détermination de la densité optique à une longueur d'onde de 492nm (référence à 620nm).

De façon intéressante, la sonde de capture utilisée est l'oligonucléotide Iag4 et la sonde de révélation est l'oligonucléotide Iag3.

Ainsi, les moyens permettent la détection qualitative ou quantitative de la présence de bactéries de type S. enterica et/ou de S. bongori, qu'il s'agisse d'une détection non spécifique au sein de l'un des groupes I, II, III, IV, V ou VI de S. enterica et de S. bongori.

Dans des conditions spécifiques de mise en oeuvre de l'étape de détection, telles qu'exposées dans l'exemple I, les amorces SS2, SS28 et la sonde SS40 permettent au contraire la détection spécifique de bactéries du groupe I de S. enterica.

D'autres caractéristiques et avantages de l'invention apparaissent dans les exemples qui suivent et dans les figures :
Figure 1 : Séquence de nucléotide d'un fragment d'ADN de 2,4 kb de la région d'invasion de Salmonella ser. Typhi.
   Les sites potentiels de liaison au ribosome sont soulignés.
Figure 2 : Pourcentage de l'activité obtenue avec différentes souches de Salmonella appartenant à différentes sérovariétés par hybridation sandwich. Sérovariétés des différents isolats de Salmonella testées :
   a: S. enterica sous-espèce enterica (I), réf.: C53
   b: S. enterica sous espèce salamae (II), réf.: 975-71
   c: S. enterica sous espèce salamae (II), réf.: 3975-83
   d: S. enterica sous espèce arizonae (IIIa), réf.: 1600 K
   e: S. enterica sous espèce arizonae (IIIa), réf.: So 20-20
   f: S. enterica sous espèce diarizonae (II), réf.: 5250-85
   g: S. enterica sous espèce diarizonae (IIIb), réf.: 8013-93
   h: S. enterica sous espèce houtenae (IV), réf.: 1357-73
   i: S. bongori, ref.: 2790-79
   k: S. enterica sous espèce indica (VI), réf.: 4355-84 - 7, 6, 5, 4, et 3: log (montant des molécules d'ADN).

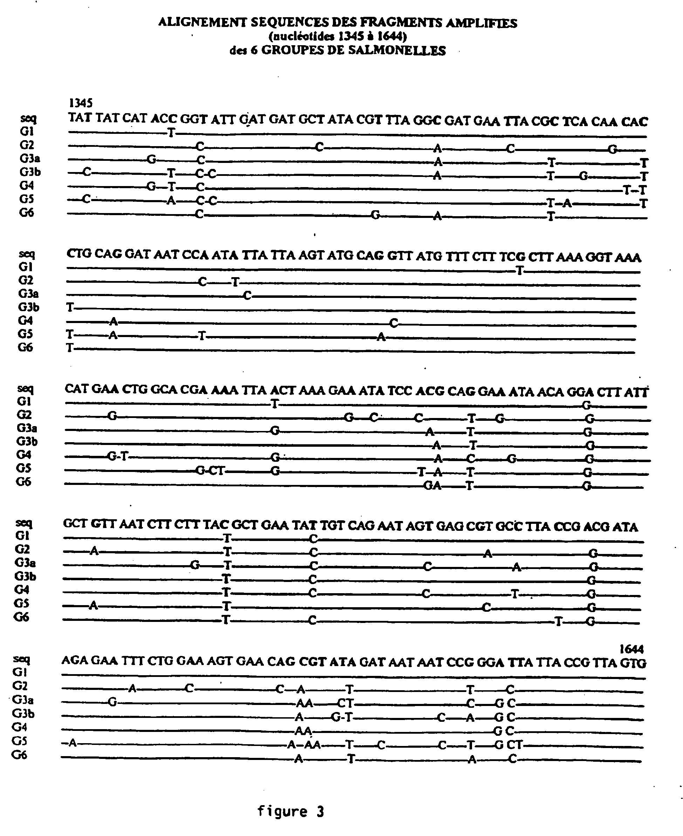

Figure 3 : Alignement des séquences des fragments amplifiés (nucléotides 1345 à 1644) des 6 groupes de Salmonelles.

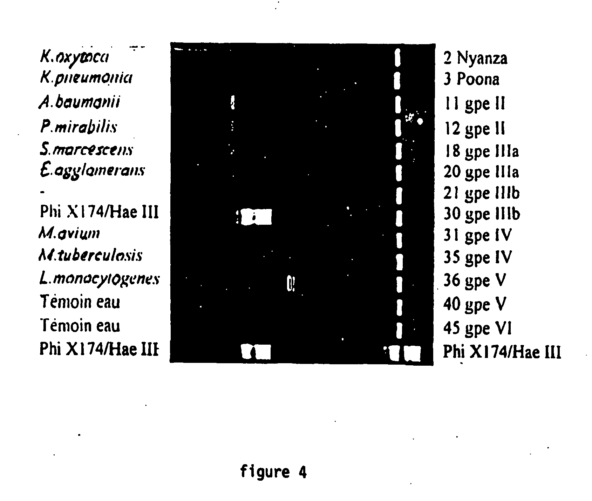

Figure 4 : Amplification grâce aux amorces Iag5 et Iag6 sur deux représentants de chacun des groupes de Salmonelles.

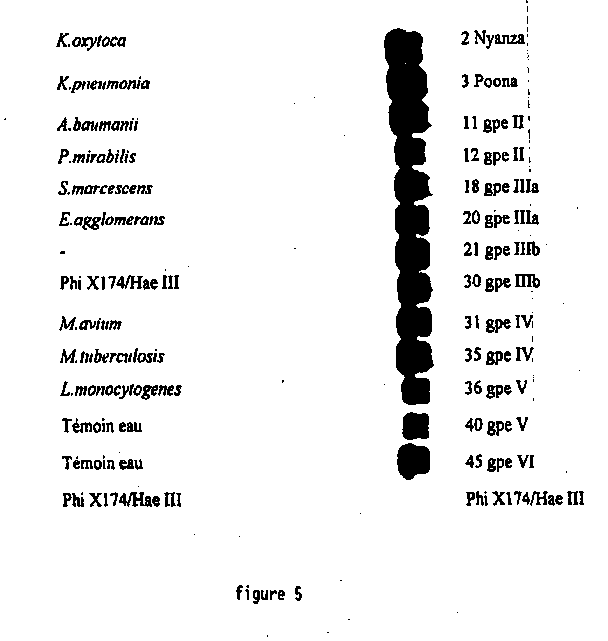

Figure 5 : Autoradiographie du Southern blot des produits amplifiés des Salmonelles.

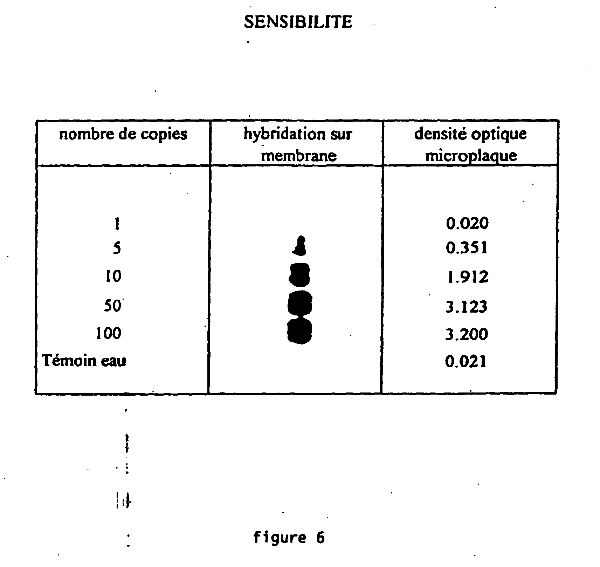

Figure 6 : Détermination du nombre minimal de molécules d'ADN chromosomique pouvant être détecté. Autoradiographie du Southern blot et hybridation sur microplaque.

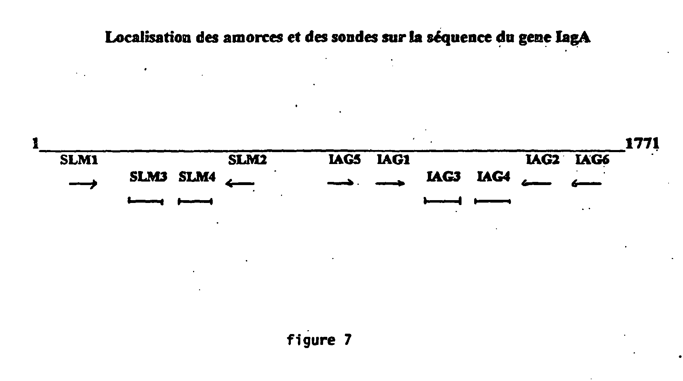

Figure 7 : Localisation des oligonucléotides sélectionnés au sein du gène IagA.

### EXEMPLE I

### CLONAGE ET SEQUENCAGE DU FRAGMENT D'ADN DE 2,4 kb

Ce fragment d'ADN a été sous-cloné en utilisant un fragment de restriction obtenu par coupure avec les enzymes HindIII, à partir de la séquence HindIII de 7,9 kb décrite dans la publication de Popoff et Dion, 1990, dans des dérivés du vecteur m13 (Messing et Vieira, 1982).

Après la réalisation de ce clonage, la méthode de dideoxy terminaison de chaine a été mise en oeuvre en utilisant la T7 DNA polymérase modifiée (Sequenase, USB Corp.) et des oligonucléotides synthétiques universels à titre d'amorces. Toutes les extrémités des fragments de restriction utilisés se chevauchaient les unes les autres. Le séquençage de l'ADN a été réalisé au moins 2 fois sur chacun des brins. La séquence de nucléotides a été analysée en utilisant le programme de Lipan et Pearson, 1985.

Comme le montre la séquence présentée à la figure 1, deux phases ouvertes de lecture sont contenues dans le fragment séquencé ; elles sont désignées par les termes iagA (abréviation de invasion associate gene) et iagB. Les deux phases ouvertes de lecture sont transcrites dans la même orientation. Le premier codon ATG (bp 97) de la phase ouverte de lecture de l'iagA, qui - est précédé par la séquence 5'-AGAGA-3', est supposé correspondre au site d'initiation de la traduction du gène iagA. Le gène iagA code pour un polypeptide comportant 553 résidus d'acides aminés avec un poids moléculaire calculé de 63026 Da. Une homologie significative a été détectée entre le domaine N-terminal de la protéine IagA et le domaine correspondant à la protéine de régulation de la transcription PhoB (24% d'identité et 52% de similitude pour une superposition de 108 acides aminés) et la protéine PhoP (25% d'identité et 69% de similitude pour 100 acide aminés alignés) de E.coli. Le codon d'initiation ATG du gène iagB (bp 1776) est également précédé par un site potentiel de liaison au ribosome (5'-AGGAAG-3'). Le gène iagB code pour un polypeptide comportant 160 acides aminés et ayant un poids moléculaire calculé de 18369 Da. La comparaison de la séquence de la protéine IagB avec les séquences traduites contenues dans la banque de données Genbank a montré une homologie significative avec la protéine IpgF (43% d'identité et 66% de similitude pour 151 acides aminés alignés).

La protéine IpgF est codée par le gène ipgF qui est situé sur le plasmide associé à la virulence de Shigella flexneri, à l'extrémité 5' du locus mxi-spa (Allaoui et al, 1993).

Les protéines mises en évidence de Salmonella enterica sous-espèce enterica sérovariété Typhi auraient donc un rôle dans l'infection par ces bactéries, et notamment dans l'adhésion et la pénétration dans les cellules.

### EXEMPLE 2

### DETECTION SPECIFIQUE DE S. ENTERICA DU GROUPE I

Un protocole de détection des sous-espèces de Salmonella par réaction de polymérisation en chaîne (PCR) a été mis au point. Un couple d'oligonucléotides utilisés comme amorce a été défini pour amplifier un fragment de 93 pb d'un gène requis pour l'invasion des cellules HeLa par S. typhi, souche Ty2. Le produit d'amplification a été analysé par une hybridation non radioactive en sandwich sur des plaques de microtitration en utilisant deux oligonucléotides différents selon la procédure décrite par Chevrier et al, 1993, Mol. Cell. Probes 7, 187-197. L'oligonucléotide de capture a été phosphorylé à son extrémité 5' et lié de façon covalente à des puits portant des groupes aminés d'une plaque de microtitration. L'oligonucléotide de détection a été aminé à son extrémité 5' puis marqué avec un biotinyl-N-hydroxy-succinimide ester. Après hybridation, les molécules hybrides ont été détectées par de l'avidine conjuguée à la phosphatase alkaline et à un substrat chromogène. Cette méthode nécessite seulement l'utilisation d'un cycler thermique et d'un lecteur de microtitration conventionnel, et peut être mise en oeuvre sur une large échelle.

### MATERIELS ET METHODES

### Souches bactériennes

Deux cent vingt huit isolats cliniques (Tableau 1) incluant S. bongori (Sambrook et al, 1989, Molecular cloning, a laboratory manual. Cold Spring Harbor Laboratory Press, Cold Spring Harbor, N.Y.), S. enterica sous espèce I(116), II(56), IIIa(11), IIIb(30), IV(5) et VI(5) et 16 souches d'Enterobactéries non-salmonelles (Tableau 2) représentant 9 genres différents ont été utilisés dans cette étude. La souche C53 de S. ser. Typhimurium a été utilisée comme contrôle positif, et la souche HB101 de E. coli a été utilisée comme contrôle négatif dans les tests de PCR.

### Extraction de l'ADN

Les souches ont été cultivées sur un milieu LB à 37°C. Afin de procéder à l'extraction rapide de l'ADN, 2 ml de la culture maintenue pendant toute la nuit ont été centrifugés et resuspendus dans 1 ml de TE (tampon 10 mM Tris-HCl à pH8 contenant 1 mM EDTA). Les cellules ont été centrifugées, le culot de centrifugation a été resuspendu dans 500 µl d'eau distillée stérile et chauffé à 100 °C pendant 10 minutes. Finalement, la solution a été centrifugée et le surnageant a été conservé pour des expérimentations en PCR.

### Amorces oligonucléotidiques et sondes

Les oligonucléotides ont été synthétisés dans un synthétiseur à ADN cyclone (Millipore-Waters) en utilisant la technologie au phosphoramidite.

Les séquences des amorces d'oligonucléotides étaient les suivantes :
SS2: 5'-CCGGGCAGATGATACCC-3' et
SS28: 5'-TAATGCTTTCCTGGTGC-3'.

La sonde oligonucléotidique de capture,
SS40: 5'-CCCGAACTATCTCGATCTGTACAATATTATCATT-3' a été phosphorylée à son extrémité 5' avec la T4 polynucléotide kinase (Boehringer) selon la description faite par Sambrook et al, 1989, (Molecular cloning, a laboratory manual. Cold Spring Harbor Laboratory Press, Cold Spring Harbor, N.Y.). La sonde de détection octadecanucléotidique SS41 (5'-GCAGGTGATAACCTTTAA-3') a été synthétisée avec une fonction amino à son extrémité 5' en utilisant la méthode au phosphoramidite sur phase solide dans un synthétiseur d'ADN Applied Biosystem 380B puis marquée avec le D-biotinyl-Σ-aminocaproic acide-N-hydroxysuccinimide ester (Boehringer) selon la description faite par Tham et al, 1990, (FEMS Microbiol. Lett. 69, 109-116). Les oligonucléotides de capture et de détection ont été tous les deux purifiés sur une colonne de dessalement rapide HR 10/10 avec le système FPLC (Pharmacia).

### Expériences de PCR

Les réactions d'amplification ont été réalisées dans un volume total de 100 µl dans un tampon 50 mM Tris-HCl pH 8,5, contenant 4 mM MgCl₂, 100 µg/ml de sérum alumine bovine, 1µM de chaque amorce, 200 µM de chaque dNTP et 1 U de Taq ADN polymérase (Amersham). Le mélange d'amplification a été recouvert avec 100 µl d'huile minérale et soumis à 10 cycles d'amplification selon la description suivante : lés échantillons ont été incubés à 94°C pendant 10 sec pour dénaturer l'ADN, à 60°C pendant 10 secondes pour apparier les amorces à l'ADN et à 72°C pendant 30 sec. pour réaliser la réaction d'extension des amorces appariées, ceci étant suivi de 30 cycles selon le protocole suivant : dénaturation à 87°C pendant 10 sec., appariement à 60°C pendant 10 sec. et extension à 72° C pendant 30 sec. Les cycles thermiques ont été réalisés dans un ensemble chauffant programmable (Thermal ractor Hybaid, UK).

Les expériences de PCR ont été réalisées avec 5µl de solution d'ADN. Chaque expérience comprenait des contrôles négatifs (5 µl de tampon TE) pour chaque groupe de 10 échantillons et à la fin de chaque série.

### Tests d'hybridation sandwich sur des bandes CovaLink NH^{®}

Deux protocoles d'hybridation non-radioactive sur des bandes CovaLink NH^{®} ont déjà été décrits par Chevrier et al (1993, Mol. Cell. Probes 7, 187-197). Dans le cas présent, une technique d'hybridation sandwich a été utilisée dans la mesure où elle permettait une meilleure sensibilité de la détection. La réaction a été réalisée sur des micropuits recouverts par de l'oligonucléotide SS40 en utilisant une procédure de liaison covalente telle que décrite en détails par Chevrier et al ou Rasmussen, S.R. et al (1991, Anal. Biochem. 198, 138-142).

Le fragment d'ADN soumis à la réaction de PCR a été dénaturé directement dans les puits en ajoutant de façon séquentielle 95 µl d'eau distillée, 5µl d'échantillon de PCR, 40 µl de sonde de détection et 14 µl de NaOH 1 N par puits. Après 10 minutes, la neutralisation a été effectuée en ajoutant 21 µl de NaH₂PO₄ 1 M contenant 1% de sarkosyle. Tous les échantillons ont été réalisés en double. Après la neutralisation, la bande a été déposée sur une surface métallique et maintenue dans un four pendant la nuit à 40°C. La concentration finale de la sonde de détection biotinylée SS41 était 0,5 nM. Pendant l'incubation dans le four, il est préférable de ne pas laisser les puits non utilisés vides, mais de les remplir avec de l'eau de façon à obtenir des échanges thermiques homogènes. Les micropuits ont été lavés 5 fois à température ambiante avec du TBS-Tw (0,15 M NaCl, 10 mM tampon Tris HCl à pH 8, 1%. Tween 20). 100 µl de conjugué alkaline phosphatase-extravidine (Sigma) dilués à 1 µg/ml dans du TBS-Tw contenant 1% de sérum albumine bovine ont été ajoutés par puits. Puis, la bande a été incubée à température ambiante pendant 1 h., lavée 5 fois avec du TBS-Tw et finalement 200 µl de diéthanolamine 1 M à pH 9,8 contenant 1mM de MgCl₂ et 1 mM de para-nitrophényl phosphate ont été ajoutés. La réaction de l'enzyme a été conduite pendant 30 minutes à 2 heures. L'absorbance a été mesurée à 405 nm en utilisant un lecteur de microplaque (Dynatech). Le signal obtenu avec la solution standard du fragment d'ADN amplifié (800 fm/puits) de S. ser. Typhimurium souche C53 a été considéré comme représentant 100% et utilisé comme référence pour chaque test d'hybridation. Les valeurs des blancs correspondent à l'absorbance moyenne mesurée dans des puits recouverts par l'oligonucléotide SS40 incubé seulement avec 0,5 nM de sonde oligonucléotidique SS41 biotinylée.

### RESULTATS

### Optimisation de la méthode

Les amorces et les sondes ont été choisies dans la séquence iagA. Différents couples d'amorces ont été testés pour optimiser la technique d'hybridation sandwich sur des microplaques CovaLink. Le couple d'amorces choisi (SS2 et SS28) a permis l'amplification spécifique de la région de 93 pb de l'ADN génomique de Salmonella. En utilisant ce couple d'amorces, on a mis en évidence qu'une concentration standard de NgCl₂ (1,5-2 mM) a conduit à un résultat d'amplification relativement inintéressant et qu'une concentration de 4 mM en MgCl₂ était nécessaire pour obtenir une amplification efficace. Des oligonucléotides internes, SS40 et SS41, ont été utilisés dans un test d'hybridation non radioactif à titre de sonde de capture et de sonde de détection respectivement.

### Spécificité de la technique

La spécificité de la méthode pour la détection des salmonelles a été évaluée avec 228 souches de Salmonella (Tableau 1) et 16 souches de bactéries hétérologues (Tableau 2). Les résulats sont résumés dans le tableau 3. Edwardsiella tarda, Klebsiella pneumoniae, des espèces d'Enterobacter et d'Acinetobacter, Pasteurella, Vibrio harveyi, Serratia marcescens et de façon plus importante des espèces de Citrobacter et tous les E. coli ont donné un signal d'hybridation inférieur à 20%. Sur la base de cette valeur, il a été conclu que toutes les souches de Salmonella appartenant à la sous-espèce I pouvaient être détectées par la présente méthode. De plus, seulement une souche (souche 3975-83) des 56 souches de la sous-espèceII et 3 souches des 11 souches de la sous-espèce IIIa ont donné un signal positif. Salmonella bongori et les souches appartenant aux sous-espèces IIIb, IV et VI n'étaient pas détectables.

### Niveau de détection de la technique avec les bactéries entières

Des dilutions au 1/10ème d'une suspension de la souche C53 de S. ser. Typhimurium (de 10⁹ à 10⁻² cellules/ml) ont été faites pour estimer le nombre minimum de bactéries qui pouvait être détecté par la PCR suivi de la technique d'hybridation non radioactive. L'ADN a été extrait de chaque suspension calibrée, en utilisant la technique d'extraction rapide par ébullition. Les résultats obtenus montrent clairement que la technique d'extraction rapide de l'ADN par la simple ébullition de la suspension avant la réaction de PCR, est une technique efficace. En effet, elle permet la détection de seulement une unité cfu.

**Tableau 1 :**

| Sous-espèces de Salmonella utilisées pour évaluer la spécificité des essais d'hybridation de l'ADN. | | | |
|---|---|---|---|
| Microorganisme testé | | N° des isolats | N° des serovars |
| *Salmonella enterica* subsp *enterica* I | | 116 | 43 |
| serovar | Adelaide | | 1 |
| | Agona | | 2 |
| | Altona | | 1 |
| | Angoda | | 1 |
| | Bardo | | 2 |
| | Blockley | | 1 |
| | Bovismorbificans | | 3 |
| | Braenderup | | 4 |
| | Brandenburg | | 1 |
| | Bredeney | | 1 |
| | Broughton | | 2 |
| | Cerro | | 1 |
| | Chester | | 1 |
| | Coeln | | 1 |
| | Concord | | 1 |
| | Dakar | | 1 |
| | Derby | | 2 |
| | Enteridis | | 28 |
| | Georgia | | 1 |
| | Hadar | | 1 |
| | Heidelberg | | 4 |
| | Ibadan | | 2 |
| | Indiana | | 1 |
| | Infantis | | 5 |
| | Lexington | | 1 |
| | London | | 1 |
| | Mbandaka | | 1 |
| | Montevideo | | 6 |
| | Moscow | | 1 |
| | Ohio | | 1 |
| | Orion | | 1 |
| | Panama | | 3 |
| | Paratyphi B | | 2 |
| | Saintpaul | | 1 |
| | Typhimurium | | 13 |
| | Typhisuis | | 1 |
| | Vaertan | | 1 |
| | Veneziana | | 1 |
| | Vinohrady | | 1 |
| | Virchow | | 10 |
| | Wien | | 1 |
| | Woodinville | | 1 |
| | Yolo | | 1 |
| *Salmonella enterica* subsp *salantae* II | | 56 | 56 |
| *Salmonella enterica* subsp *arizonae* IIIa | | 11 | 29 |
| *Salmonella enterica* subsp *diarizonae* IIIb | | 30 | 5 |
| *Salmonella enterica* subsp *houtenae* IV | | 5 | 5 |
| *Salmonella enterica* subsp *indica* VI | | 5 | 5 |
| *Salmonella bongori* | | 5 | 5 |
| (initialement *S. enterica* subsp. *bongori* V) | | | |

**Tableau 2 :**

| Bactéries hétérologues utilisées dans le test d'hybridation de l'ADN | | |
|---|---|---|
| Genre | Espèces | Nombre d'isolats |
| *Escherichia* | *coli* | 4 |
| *Edwarsiella* | *tarda* | 1 |
| *Citrobacter* | *amalonaticus* | 1 |
| | *freundii* | 1 |
| *Klebsiella* | *pneumoniae* | 1 |
| *Enterobacter* | *agglomerans* | 1 |
| | *asburiae* | 1 |
| | *hormoechei* | 1 |
| *Pasteurella* | *multocida* | 1 |
| *Acinetobacter* | *lwoffii* | 1 |
| | *haemolyticus* | 1 |
| *Vibrio* | *harveyi* | 1 |
| *Serratia* | *marcescens* | 1 |

**Tableau 3 :**

| Souches cliniques de bactéries et témoins testés dans un essai d'hybridation Sandwich | | | | | | | | | |
|---|---|---|---|---|---|---|---|---|---|
| | *S.enterica* subsp. *enterica* | *S. enterica* subsp. *salamae* | *S. enterica* subsp. *arizonae* | *S. enterica* subsp *diarizonae* | *S. enterica* subsp. *houtenae* | *S. enterica* subsp. *indica* | *S. bongori* | Non *Salmonella* | Témoin sans ADN |
| *activité (%)* | | | | | | | | | |
| 100%-20% | 116 | 1 | 3 | 0 | 0 | 0 | 0 | 0 | 0 |
| 19% | 0 | 51 | 8 | 12 | 4 | 4 | 5 | 9 | 0 |
| > blanc | | | | | | | | | |
| < blanc | 0 | 4 | 0 | 18 | 1 | 1 | 0 | 7 | 23 |
| Total | 116 | 56 | 11 | 30 | 5 | 5 | 5 | 16 | 23 |

### Hybridation quantitative avec l'ADN génomique purifié

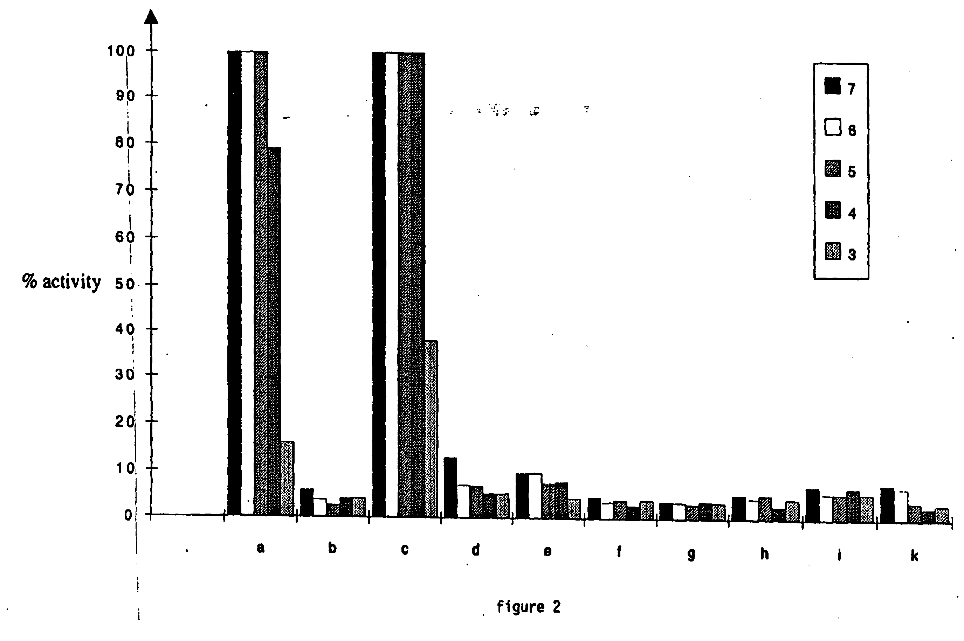

La procédure d'hybridation non-radioactive utilisée dans les tests rapportés ici peut être facilement mise en oeuvre dans des études quantitatives. Pour comparer les signaux d'hybridation obtenus avec différentes souches de Salmonella, l'ADN a été extrait de 10 souches représentant les 6 sous-espèces de Salmonella enterica et l'espèce Salmonella bongori, puis des quantités calibrées d'ADN ont été soumises à des réactions de PCR suivies par une hybridation sandwich. Les résultats sont rapportés à la figure 2. Il a été démontré que le signal d'hybridation obtenu avec 10⁷ molécules d'ADN de Salmonella bongori ou des sous-espèces II, IIIa, IIIb, IV et VI de Salmonella enterica est plus faible que le signal d'hybridation observé avec 10³ molécules d'ADN des souches de la sous-espèce I. Cependant, il est important de noter que l'isolat 3975-83 (sous-espèce II) a donné le même signal d'hybridation que les souches appartenant aux sous-espèces I.

### DISCUSSION

L'amplification par PCR permet une détection très sensible de séquences d'ADN spécifique. La sensibilité de l'amplification dépend essentiellement du nombre de copies de l'ADN cible, de la pureté de l'échantillon à analyser, de la méthode d'extraction de l'ADN, et de la sensibilité de la méthode utilisée pour détecter les produits de PCR. La visualisation des produits de PCR par une coloration au bromure d'éthidium dans un gel d'électrophorèse, n'est pas compatible avec l'utilisation en routine de la technique et n'est pas suffisamment sensible. La sensibilité peut être améliorée par l'utilisation de la PCR double ou de sondes d'ADN avec une hybridation en Dot blot ou en Southern blot. Cependant la PCR double est très sensible à la contamination par de l'ADN et les techniques d'hybridation Dot blot ou Southern blot ne sont pas appropriées pour l'automatisation. L'hybridation sur microplaque offre donc une technique appropriée pour la détection et la quantification de fragments amplifiés par PCR. L'attachement covalent simple des acides nucléiques à des micropuits représente une variante intéressante à l'adsorption passive et une amélioration importante pour la détection de fragments amplifiés par PCR sur des micropuits.

Il est connu que les souches de Salmonella provoquant des infections chez l'homme appartiennent essentiellement à la sous-espèce I. En effet, plus de 95% des isolats cliniques chez l'humain appartiennent à cette sous-espèce (Rowe, B., 1987, Salmonella surveillance. Reports received from centers participating in the WHO programme. World Health organization London). De plus, en 1991, le "Centre National d'Etudes Vétérinaires et Alimentaires" de Paris (France) rapportait [Corbion, B. et al, 1991, Inventory of Salmonella] que dans les années précédentes, la plupart des souches isolées chez l'animal dans la nourriture ou dans l'environnement en 1988 et 1989 (c'est à dire, 18832 souches) appartient à la sous-espèce I (99,2%).

Les résultats rapportés ici ont permis de définir une méthode fondée sur l'amplification par PCR pour la détection de souches pathogènes de Salmonella. Un couple d'amorces, SS2 et SS28, et un couple de sondes, SS40 et SS41 ont été sélectionnés à partir d'un gène nécessaire à l'invasion des cellules HeLa par Salmonella ser. Typhi souche Ty2. En utilisant la combinaison entre la technique de PCR et l'hybridation sandwich non radioactive sur microplaque, toutes les Salmonella de la sous-espèce I ont été détectées.

La limite de détection était inférieure à un seuil représenté par 10 cellules par tube de PCR, ce qui est conforme aux résultats obtenus par d'autres techniques de PCR similaires. Etant donné la parenté des acides nucléiques entre les membres des enterobactéries, il était important de contrôler la spécificité de ces nouvelles amorces et sondes avec les genres d'enterobactéries les plus susceptibles de conduire à des réactions de type "faux-positif". Des résultats obtenus, on peut conclure qu'aucune réaction de faux-positif ne peut avoir lieu lorsque les conditions de la PCR et de l'hybridation décrites précédemment sont suivies.

Il est intéressant de noter que la souche Salmonella 3975-83 (sous-espèce II) présentait un signal d'hybridation identique à celui obtenu avec les isolats appartenant à la sous-espèce I. Cette souche a été isolée en 1983 à partir des selles d'un patient humain en Grande-Bretagne. Sur la base des caractéristiques biochimiques, cette nouvelle sérovariété a été classée dans la sous-espèce II mais a été considérée comme une souche atypique puisque sa présence dans la gélatinase n'a pas été détectée (Le Minor, L.:et al, 1984, Supplément No. XXVII, 1983, to Kauffmann-White Scheme, Ann. Microbiol. (Institut Pasteur) 135 B, 45-51). A la lumière des résultats rapportés ici, la position taxonomique de la souche 3975-83 devrait être ré-examinée en utilisant la technique,d'hybridation ADN-ADN.

Les données présentées ici indiquent que la méthode d'hybridation basée sur l'utilisation d'un gène nécessaire à l'invasion des cellules HeLa par Salmonella ser. Typhi souche Ty2 peut distinguer les souches de la sous-espèce I des Salmonella des autres bactéries enteriques, y compris E. coli. L'hybridation non-radioactive sur une microplaque Covalink NH est sensible et appropriée pour l'analyse d'un grand nombre d'échantillons.

### EXEMPLE 3 : DETECTION DE L'ADN DE SALMONELLA AMPLIFIE PAR HYBRIDATION SANDWICH

### Séquence des oligonucléotides

Les fragments d'ADN choisis sont les suivants (voir position sur la séquence de la figure 1) :

**Partie C-terminale**

| | | position |
|---|---|---|
| Iag1: | 5'-TA TTA AGT ATG CAG GTT ATG - 3' | 1424-1443 |
| Iag2: | 5'-AGA GAA TTT CTG GAA AGT GAA- 3 ' | 1585-1605 |
| Iag3: | 5'-ATA TCC ACG CAG GAA ATA ACA GGA CTT -3' | 1495-1521 |
| Iag4: | 5'-GAG CGT GCC TTA CCG ACG ATA-3' | 1564-1584 |
| Iag5: | 5'-GCA GGG ATC ACT AAG CTG TG-3' | 1318-1337 |
| Iag6: | 5'-CGT GGG CAA CCA GCA CTA ACG-3' | 1637-1657 |
| | | |
| Slm1: | 5'-CG GGT TAA AGG TTA TCA CCT-3' | 709-728 |
| Slm2: | 5'-AG CAT GGC GCA AAT GGG-3' | 1014-1031 |
| Slm3: | 5'-GCA CCA GGA AAG CAT TAA GTT GAT AGA | |
| ACA C-3' | | 732-762 |
| Slm4: | 5'-CTT CGC TGG GAC ACA AAG CA-3' | 823-842 |

Préférentiellement, le couple d'amorces Iag5 (sens) et Iag6 (antisens) dirige l'amplification d'un fragment de 340bp, le couple Slm1 (sens) et Slm2 (antisens) dirige l'amplification d'un fragment de 323bp (figure 3).

La figure 4 montre l'efficacité de l'amplification du couple d'amorces Iag5 et Iag6 sur 2 représentants de chacun des groupes de Salmonelles.

### Procédé de détection.

Un format de détection par hybridation sandwich a été utilisé.

Deux oligonucléotides s'hybrident simultanément au fragment amplifié dénaturé. L'un d'eux appelé sonde de capture est fixé de façon passive (mais peut aussi être fixé de façon covalente) à la surface d'un puits de plaque de microtitration 96 puits. L'autre appelé sonde de révélation est marqué par un élément facile à mettre en évidence. La sonde de révélation est libre dans le tampon d'hybridation.

Les sondes de capture et de révélation sont complémentaires de 2 régions différentes situées à l'intérieur du fragment amplifié.

La sonde de détection dans le cas ici décrit, est liée à un marqueur enzymatique notamment une peroxydase et va servir de sonde de révélation. C'est le cas préférentiellement des oligonucléoties Iag3 et Slm3. D'autres oligonucléotides peuvent être fixés à un support solide de type microplaque, un support articulaire ou membranaire et servir de sonde de capture, ceci particulièrement pour les oligonucléotides Iag4 et Slm4.

### Conditions expérimentales:

### 1) Préparation de l'ADN des Salmonelles

Par la méthode d'ébullition en présence de Chelex (Chelex 6%, SDS 0.1%, NP40 1%, Tween 20 1%), on obtient les séquences d'ADN. Ce réactif est commercialisé par Biorad et utilisé selon le protocole du fabricant (ref. Walsh et al. 1991. BioTechniques 10 : 506-513).

### 2) Amplification

Selon la méthode initialement décrite par Saiki et telle qu'exposée par exemple dans le brevet européen EP 0201184.

La PCR est réalisée en utilisant le mélange réactionnel suivant :
50 mM KCl
10mM Tris-HCl pH 8.3
1,5mM MgCl₂
125 µM désoxyribonucléotides (dCTP, dATP, dGTP)
250 µM d'UTP
25 pmoles de chacune des amorces
10ng ADN
1 unité d'Uracyl N Glycosylase
1 unité de Taq polymerase.

Le mélange réactionnel a été réalisé en utilisant 10µl de la solution contenant l'ADN à amplifier sous un volume de 100µl. Le dUTP et l'UNG sont utilisés en système de décontamination (Brevet Life Technologies European Patent Application 0 401 037). Le thermocycler utilisé est le 9600 de Perkin Elmer.

Après une incubation à 50°C pendant 2 min pour permettre l'action de l'UNG et une dénaturation à 95°C pendant 5min, les cycles de température utilisés sont les suivants :
- 5 cycles (95°C 15 sec, 50°C 15 sec, 72°C 15 sec)
- 35 cycles (95°C 15 sec, 57°C 15 sec, 72°C 15 sec)

### 3) Visualisation de la réaction d'amplification

### 3-1) Marquage de la sonde de révélation

Les sondes sont marquées à la peroxydase de raifort (ref. PCR protocols : a guide to methodes and application; Academic press (1990), 15, p4513-4534) et l'activité de l'enzyme est révélée en colorimétrie.

### 3-2) Gel d'agarose coloré au BET et hybridation sur membrane

Après amplification, 10µl du produit d'amplification sont déposés sur gel d'agarose et l'ADN est transféré sur membrane selon les techniques classiques (Maniatis). La membrane est préhybridée, 30 min à 68°C en tampon d'hybridation (10X Denhart, 6X SSC, 0,1%SDS) puis hybridée à 42°C pendant 3h avec 60 ng de sonde par ml de tampon d'hybridation.

Un lavage est ensuite effectué selon les étapes suivantes :
- 2 fois 10 min en 2 X SSC -0,1%SDS à température ambiante,
- 1 fois 30 min en 0,1 X SSC - 0,1% SDS à 42°C,
- 2 fois 10 min en 2 X SSC à température ambiante.

Révélation : La membrane est épongée entre deux feuilles de papier absorbant (papier Whatman 3MM) et posée dans un bac propre et sec.

Le réactif de détection Amersham (réactif de détection ECL RPN 2105) est préparé extemporanément volume à volume ; 30 ml de volume total pour une membrane 5 x 8 cm. Une cassette pour autoradiographie est obtenue en fixant une feuille de papier absorbant (papier Whatman 3MM) au fond. Toutes ces étapes peuvent se faire à la lumière. Ensuite en chambre noire.

On baigne la membrane dans le réactif de détection pendant 1 min, côté ADN vers le haut, on égoutte rapidement la membrane, on la place dans la cassette, côté ADN vers le haut, on pose dessus une feuille en plastique transparent (sinon la membrane se colle sur le film) :et on met un film radiographique par dessus (film X-OMAT KODAK).

L'exposition est réalisée pendant 30 min à température ambiante puis le film est développé par les techniques de révélation classique (révélateur, eau, fixateur).

### 3-3) microplaque

### 3-3-1) Coating de l'oligonucléotide de capture

Il peut se faire par adsorption (Cook et al, NAR, 16: 4077-4095 (1988) ou couplage covalent (Rasmussen, S.R. et al, 1991. Analytical Biochemistry 198, 138-142).

### 3-3-2)Hybridation en microplaque et lecture

10 µl du produit d'amplification ont été dénaturés par addition volume à volume d'une solution 200mM NaOH, 40mM EDTA.

Les microplaques dont la surface des puits est revêtue de la sonde de capture, ont été préhybridées dans un tampon d'hybridation contenant 10XDenhart, 6XSSC, 0,1% SDS.

Puis la microplaque a été vidée et chacune des cupules a reçu 200µl de tampon d'hybridation renfermant le fragment amplifié dénaturé et la sonde de révélation à la concentration de 10ng/µl. L'incubation a eu lieu pendant une heure à 37°C et sous agitation.

Après lavage (solution de lavage 10X :100mM Tris, 3M NaCl, 1% Tween 20, pH 7,4), l'activité de la peroxydase liée à la sonde a été détectée en colorimétrie en présence d'un substrat coloré.

Pour ce faire, 200µl d'une solution 40mM citrate trisodique, 0,03% H₂O 30%, 7,5mg/ml d'orthophenylenediamine (OPD) ont été distribués dans chacun des puits. La microplaque a été incubée 30min à l'obscurité et à 37°C. 50µl/puits d'une solution 4N H₂SO₄ ont été ajoutés pour bloquer la réaction.

La densité optique a été déterminée à une longueur d'onde de 492nm (référence à 620nm).

### 4) Séquençage des produits PCR et alignement manuel des séquences.

Selon les techniques classiques, en utilisant par exemple un automate "373 DNA sequencer" d'Applied Biosystem et le kit "dye terminator" d'Applied.

### Résultats

Le modèle exemplifié est préférentiellement le système d'oligonucléotides suivant :
Iag5 amorce sens-Iag6 amorce antisens
Iag3 sonde de révélation et Iag4 sonde de capture.
   (il est à noter que Iag4 peut tout aussi bien être marquée et être utilisée en sonde de révélation).

### Etude de Spécificité

Elle a porté sur l'ensemble des souches bactériennes listées dans les Tableaux 4 et 5.

L'amplification de l'ADN extrait des 45 souches de Salmonelles testées a généré un fragment de la taille attendue (cf. Fig. 5). Les Southern blots de l'ensemble des produits amplifiés ont été hybridés avec la sonde oligonucléotidique interne Iag 3 marquée à la peroxydase. Aucune des souches non salmonelles n'a donné lieu à une hybridation avec une sonde peroxydase réalisée sur membrane selon le protocole décrit plus haut.

Les mêmes produits d'amplification ont été testés en format microplaque.

Le cut-off a été arbitrairement fixé à 0.050. Tous les représentants de chacun des groupes de Salmonelles donnent une valeur de densité optique supérieure à 0.050 (tableau 6).

### Sensibilité

Pour déterminer le nombre minimal de molécules d'ADN chromosomique de salmonelles pouvant être détecté, une gamme de dilution d'ADN chromosomique purifié a été amplifiée. 5 molécules sont visibles sur l'autoradiographie du southern blot et détectées en hybridation microplaque : la valeur obtenue en colorimétrie est supérieure au Cut-Off (Figure 6).

Les oligonucléotides sélectionnés pour la réalisation de cet exemple ont été localisés sur la séquence du gène IagA (figure 7).

### SOUCHES DE SALMONELLES ETUDIEES

**TABLEAU 4**

| **N°** | **Souches** | **Sérotype** | **Groupe** |
|---|---|---|---|
| 1 | *Salmonella Marseille* | | I |
| 2 | *Salmonella Nyanza* | | I |
| 3 | *Salmonella Poona* | | I |
| 4 | *Salmonella Kampala* | | I |
| 5 | *Salmonella Taksony* | | I |
| 6 | *Salmonella Teshie* | | I |
| 7 | *Salmonella Indiana* | | I |
| 8 | *Salmonella enteritidis* | | I |
| 9 | *Salmonella Kentucky* | | I |
| 10 | *Salmonella Napoli* | | I |
| 11 | | 841 11:a:d:en 215 | II |
| 12 | | 1703 K 41: 2: 15 | II |
| 13 | | 950-7143:d:z39 | II |
| 14 | | 10-65 44 : 24, 223 : - | II |
| 15 | | 3209-81 45: z 23 | II |
| 16 | | 5331 / 86 62: z 29 : - | IIIa |
| 17 | | 3064-4 / 252 41: k : - | IIIa |
| 18 | | 594-54 38: z 54: - | IIIa |
| 19 | | 1694 cdai 426 63: z 4, z32: - | IIIa |
| 20 | | So 50 / 16 62: f, z 51: - | IIIa |
| 21 | | 5251-85 58: r : z 53 | IIIb |
| 22 | | 1758-76 6,14 : z 10 : enx 215 | IIIb |
| 23 | | 453-68 16 : liv : z53 | IIIb |
| 24 | | 4305-57 16 : li(v) : z 35 | IIIb |
| 25 | | 1698-75 11 : liv : z | IIIb |
| 26 | | 8275-94 47 : r : enx 215 | IIIb |
| 27 | | 8283-94 53 : z 10 : z | IIIb |
| 28 | | cdc 456-5 / 93 40 : i : 1, 5, 7 | IIIb |
| 29 | | 8284-94 60: i : z | IIIb |
| 30 | | 1693 K 38 : k : z 55 | IIIb |
| 31 | | 1707 48 : f: z 51 :- | IV |
| 32 | | 7231 / 89 45 : z 36. z 38 | IV |
| 33 | | 6887 / 60 48 : f, z 51 : - | IV |
| 34 | | 1357 /73 43 : z4, z 24 : - | IV |
| 35 | | 1550 K 16 :z 4, z 23 : - | IV |
| 36 | *Salmonella Bongor* | 261 -66 48 : z35 : - | V |
| 37 | *Salmonella Camdeni* | 2022 - 77 44 : r : - | V |
| 38 | | 4985 - 85 48 : z 39 : - | V |
| 39 | | 7688 - 9166 : z 39 : - | V |
| 40 | | 1387 - 7340 : a : | V |
| 41 | | 1941 - 77 6.7 : z 41: 1,7 | VI |
| 42 | | · 1449 K45 : a enx | VI |
| 43 | | 4355 · 84 1,6. 14,25 : a :e,n,x | VI |
| 44 | | 1711 K 11 : b: enx | VI |
| 45 | | 1688 K 1,6. 14,25 : Z 10 : 1,12,7 | VI |

### SOUCHES NON SALMONELLES

**TABLEAU 5**

| **N°** | **Nom** | **Identification** |
|---|---|---|
| 1 | *Klebsiella oxytoca* | 0059 SDP |
| 2 | *Klebsiella pneumoniae* | 0054 SDP |
| 3 | *Acinetobacter baumanii* | 0033 SDP |
| 4 | *Proteus mirabilis* | RP402 |
| 5 | *Serratia marcescens* | 0042 SDP |
| 6 | *Enterobacter agglomerans* | 0067 SDP |
| 7 | *Citrobacter diversus* | 0068 SDP |
| 8 | *Pseudomonas aeruginosa* | 0011 SDP |
| 9 | *Enterobacter aerogenes* | 0066 SDP |
| 10 | *Escherichia coli* | 0131 SDP |
| 11 | *Enterocoque faecalis* | 76117 |
| 12 | *Proteus mirabilis* | AP03 |
| 13 | *Enterocoque faecalis* | 76117 |
| 14 | *Enterobacter cloacae* | 0060 SDP |
| 15 | *Mycobacterium avium* | *6* |
| 16 | *Mycobacterium tuberculosis* | H 37 RV |
| 17 | *Listeria monocytogenes* | 1/2 LG3 |

### DETECTION SUR MICROPLAQUE

**TABLEAU 6**

| **ECHANTILLONS** | **DO à 420 nm** |
|---|---|
| 2 Nyanza gpe I | 3.029 |
| 3 Poona gpe I | 3.103 |
| 11 gpe II | 3.155 |
| 12 gpe II | 0.751 |
| 18 gpe III a | 3.139 |
| 20 gpe III a | 3.068 |
| 21 gpe III b | 3.161 |
| 30 gpe III b | 3.201 |
| 31 gpe IV | 0.272 |
| 35 gpe IV | 0.527 |
| 36 gpe V | 1.868 |
| 40 gpe V | 3.347 |
| 45 gpe VI | 0.900 |
| *Klebsiella oxytoca* | 0.022 |
| *Klebsiella pneumoniae* | 0.017 |
| *Acinetobacter baumanii* | 0.024 |
| *Proteus mirabilis* | 0.019 |
| *Serratia marcescens* | 0.019 |
| *Enterobacter agglomerans* | 0.023 |
| *Mycobacterium avium n° 6* | 0.025 |
| *Mycobacterium tuberculosis H 37 RV* | 0.020 |
| *Listeria monocytogenes 1*/*2 LG3* | 0.015 |
| Témoin eau | 0.018 |
| Témoin eau | 0.022 |

## Revendications

1. Couple d'amorces oligonucléotidiques **caractérisé en ce qu'**il consiste en deux oligonucléotides d'au moins 9 nucléotides hybridant dans des conditions stringentes avec la séquence nucléotidique correspondant à la partie C-terminale du gène iagA définie entre les positions 1318 et 1755 de la séquence iagA représentée à la Figure 1, ledit couple étant capable d'amplifier une séquence d'ADN ou d'ADNc de *Salmonella enterica* et de *Salmonella bongori* comprise dans la séquence iagA.

2. Couple d'amorces selon la revendication 1, **caractérisé en ce que** les conditions d'hybridation stringentes sont 50°C, dans le mélange réactionnel suivant : 50 mM KCl, 10 mM Tris-Hcl pH 8,3, 1,5 mM MgCl₂. 125 µM de dCTP, dATP et dGTP, 250 µM de dUTP, 25 pmole de chacune des amorces, 1 unité d'Uracyl N Glycosylase, et 1 U de Taq.

3. Kit pour la détection de la présence de *Salmonella enterica* et/ou de *Salmonella bongori* dans des échantillons biologiques, comprenant :
- un couple d'amorces oligonucléotidiques selon la revendication 1 ou 2,
- une sonde pour la détection des fragments amplifiés, et
- les réactifs nécessaires à la réalisation de la réaction d'amplification.

4. Utilisation d'un couple d'amorces selon la revendication 1 pour l'amplification de séquences de nucléotides de bactéries du genre *Salmonella* espèces *enterica et bongori.*

5. Utilisation d'un couple d'amorces oligonucléotidiques selon la revendication 1, pour la détection après amplification de l'ADN génomique ou de l'ADNc de bactéries du genre *Salmonella* espèces *enterica et bongori.*

6. Utilisation selon la revendication 4 ou 5, dans laquelle les conditions d'hybridation stringentes sont 50°C, dans le mélange réactionnel suivant : 50 mM KCl, 10 mM Tris-Hcl pH 8,3, 1,5 mM MgCl₂, 125 µM de dCTP, dATP et dGTP, 250 µM de dUTP, 25 pmole de chacune des amorces, 1 unité d'Uracyl N Glycosylase, et 1 U de Taq.

## Claims

1. Couple of oligonucleotide primers **characterized by** consisting of two oligonucleotides of at least 9 nucleotides hybridizing under stringent conditions with the nucleotide sequence which corresponds to the C-terminal end of the iagA gene defined between positions 1318 and 1755 of the iagA sequence shown in Figure 1, said couple being capable of amplifying a DNA or a cDNA sequence of *Salmonella enterica* and of *Salmonella bongori* contained in the iagA sequence.

2. Couple of primers according to claim 1, **characterized by** the fact that the conditions of stringent hybridization is 50°C in the following reactive mixture: 50 mM KCl, 10 mM Tris-Hcl pH 8,3, 1,5 mM MgCl₂, 125 µM of dCTP, dATP and dGTP, 250 µM of dUTP, 25 pmole of each of the primers, 1 unit of Uracil-N-Glycosylase, and 1 U of Taq.

3. Kit for the detection of *Salmonella enterica* and/or *Salmonella bongori* in biological samples, comprising:
- a couple of oligonucleotide primers according to claim 1 or 2,
- a probe for the detection of amplified fragments; and
- the necessary reagents to achieve the amplification reaction.

4. Use of a couple of primers according to claim 1 for the amplification of bacteria nucleotide sequences of the *Salmonella* genus, *enterica* and *bongori* species.

5. Usage of a couple of oligonucleotide primers according to claim 1 for the detection, after amplification of genomic DNA or cDNA, of bacteria of the *Salmonella* genus, *enterica* and *bongori* species.

6. Usage according to claims 4 or 5, in which conditions of stringent hybridization are 50°C in the following reactive mixture: 50 mM KCl, 10 mM Tris-Hcl pH 8,3, 1,5 mM MgCl₂, 125 µM of dCTP, dATP et dGTP, 250 µM of dUTP, 25 pmole of each of the primers, 1 unit of Uracil-N-Glycosylase, and 1 U of Taq.

## Patentansprüche

1. Oligonucleotid-Primer-Paar, **dadurch gekennzeichnet, dass** es aus zwei Oligonucleotiden mit mindestens 9 Nucleotiden besteht, die unter stringenten Bedingungen mit der Nucleotidsequenz hybridisieren, welche dem C-terminalen Teil des Gens iagA entspricht, welcher zwischen den Positionen 1318 und 1755 der in Figur 1 dargestellten Sequenz iagA definiert ist, wobei das Paar in der Lage ist, eine DNA- oder cDNA-Sequenz von *Salmonella enterica* und von *Salmonella bongori,* die in der Sequenz iagA eingeschlossen ist, zu amplifizieren.

2. Primer-Paar nach Anspruch 1, **dadurch gekennzeichnet, dass** die stringenten Hybridisierungsbedingungen 50 °C in der folgenden Reaktionsmischung sind:
50 mM KCl, 10 mM Tris-HCl pH 8,3, 1,5 mM MgCl₂, 125 µM dCTP, dATP und dGTP, 250 µM dUTP, 25 pMol jedes Primers, eine Einheit Uracyl-N-Glycosylase und 1 E Taq.

3. Kit für den Nachweis der Anwesenheit von *Salmonella enterica* und/oder von *Salmonella bongori* in biologischen Proben, umfassend:
- ein Oligonucleotid-Primer-Paar nach Anspruch 1 oder 2,
- eine Sonde für den Nachweis von amplifizierten Fragmenten und
- die notwendigen Reagenzien zur Durchführung der Amplifikationsreaktion.

4. Verwendung eines Primer-Paars nach Anspruch 1 für die Amplifikation von Nucleotidsequenzen von Bakterien der Gattung *Salmonella,* Spezies *enterica und bongori.*

5. Verwendung eines Oligonucleotid-Primer-Paars nach Anspruch 1 für den Nachweis nach Amplifikation von genomischer DNA oder cDNA von Bakterien der Gattung *Salmonella,* Spezies *enterica und bongori.*

6. Verwendung nach Anspruch 4 oder 5, bei der die stringenten Hybridisierungsbedingungen 50 °C in der folgenden Reaktionsmischung sind: 50 mM KCl, 10 mM Tris-HCl pH 8,3, 1,5 mM MgCl₂, 125 µM dCTP, dATP und dGTP, 250 µM dUTP, 25 pMol jedes Primers, eine Einheit Uracyl-N-Glycosylase und 1 E Taq.
